(19) Europäisches Patentamt
European Patent Office
Office européen des brevets

(11) EP 0 841 330 B1

(12) FASCICULE DE BREVET EUROPEEN

(45) Date de publication et mention de la délivrance du brevet: **27.06.2001 Bulletin 2001/26**

(21) Numéro de dépôt: **97402655.1**

(22) Date de dépôt: **06.11.1997**

(51) Int Cl.[7]: **C07D 263/58**, C07D 265/36, C07D 277/68, C07D 279/16, A61K 31/42, A61K 31/425, A61K 31/535, A61K 31/54

# (54) Nouveaux dérivés aminométhyl hétérocycliques, leur procédé de préparation et les compositions pharmaceutiques qui les contiennent

Aminomethylheterozyclischederivate, Verfahren zur ihrer Herstellung und diese enthaltende pharmazeutische Zusammensetzungen

Aminomethyl heterocyclic derivatives, process of their preparation and pharmaceutical compositions containing them

(84) Etats contractants désignés:
**AT BE CH DE DK ES FI FR GB GR IE IT LI LU NL PT SE**

(30) Priorité: **08.11.1996 FR 9613652**

(43) Date de publication de la demande:
**13.05.1998 Bulletin 1998/20**

(73) Titulaire: **ADIR ET COMPAGNIE**
**92415 Courbevoie Cédex (FR)**

(72) Inventeurs:
- **Lesieur, Daniel**
  **59147 Gondecourt (FR)**
- **Carato, Pascal**
  **03600 Commentry (FR)**
- **Bonte, Jean-Paul**
  **59290 Wasquehal (FR)**
- **Depreux, Patrick**
  **59280 Armentieres (FR)**
- **Caignard, Daniel-Henri**
  **78230 Le Pecq (FR)**
- **Millan, Mark**
  **78230 Le Pecq (FR)**
- **Newman-Tancredi, Adrian**
  **78230 Le Pecq (FR)**
- **Renard, Pierre**
  **78000 Versailles (FR)**
- **Rettori, Marie-Claire**
  **92400 Courbevoie (FR)**

(56) Documents cités:
**EP-A- 0 281 309**
**EP-A- 0 478 446**
**EP-A- 0 488 008**
**EP-A- 0 506 539**
**EP-A- 0 613 898**
**EP-A- 0 673 933**
**EP-A- 0 673 934**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 841 330 B1

Printed by Jouve, 75001 PARIS (FR)

## Description

La présente invention concerne de nouveaux dérivés amino méthyl hétérocycliques, leur procédé de préparation et les compositions pharmaceutiques qui les contiennent.

De nombreuses amines alkyl hétérocycliques à motif benzoxazolinone, benzothiazolinone ou benzoxazinone ont déjà été décrites.

Le brevet européen EP 0 110781 décrit des (amino-2 éthyl)-6 benzoxazolinones en tant qu'hypnotiques et agents de traitement de l'insuffisance cardiaque. La demande de brevet européen EP 0 281309 décrit des composés de structure pipérazinoéthyl ou butyl benzoxazolinoniques et benzothiazolinoniques intéressants en tant qu'antipsychotiques.

Le brevet français FR 2035749 décrit des aminoalkyl benzoxazinones intéressantes pour le traitement des troubles du système nerveux central.

La publication "Il farmaco" 89, 44 (1), 77-88 décrit des aryl pipérazinobutyl benzoxazolinones ainsi que leurs propriétés essentiellement analgésiques.

La demande de brevet EP 0 223674 décrit des acyl-7 benzoxazinones et leurs dérivés comme possédant des propriétés antiathérosclèreuses et normolipémiantes.

La demande de brevet EP 0 478446 décrit des amines éthyl et butyl hétérocycliques qui possèdent la propriété de se fixer avec une très haute affinité sur les récepteurs sérotoninergiques 5-$HT_{1A}$.

Les demandes de brevet EP 0 673 933 et EP 0 673 934 décrivent des dérivés aminoalkyl benzoxazolinones et benzothiazolinones qui ont une très haute affinité sélective pour les récepteurs sigma.

La demanderesse a présentement découvert de nouveaux dérivés amino méthyl hétérocycliques plus particulièrement amino méthyl benzoxazolinoniques et benzothiazolinoniques qui contrairement aux dérivés de l'art antérieur et de façon tout à fait surprenante n'ont plus qu'une faible affinité pour les récepteurs 5-$HT_{1A}$ et $D_2$. Parallèlement ces nouveaux dérivés possèdent une excellente affinité pour les récepteurs $D_4$ alors qu'une comparaison effectuée sur les dérivés mentionnés dans les documents de l'art antérieur a en effet établi que ces derniers n'avaient aucune affinité pour ces récepteurs $D_4$.

Les récepteurs $D_4$ sont localisés dans les structures cortico-limbiques (cortex frontal, nucleus accumbens et hippocampe) impliquées dans le contrôle de l'humeur et de la mémoire (Bloom and Kupfer, in Psychopharmacology "The fourth generation of progress" Raven Press, New York 1995 ; Meador-Woodruff et al., "Dopamine receptor mRNA expression in human striatum and neocortex". Neuropsychopharmacology, 1996 ; 15 : 17-29). Dans ces structures, une sous-population est localisée sur les neurones de type gabaergique, qui jouent également un rôle clé dans la modulation de l'humeur et des fonctions cognitives (Bloom and Kupfer, 1995 (déjà cité)) ; Mrzkjak et al., "Localisation of dopamine $D_4$ receptors in GABAergic neurons of the primate brain". Nature, 1996 ; 381 : 245-248). Certaines études ont montré une augmentation de la densité des récepteurs $D_4$ chez les patients psychotiques tandis que la clozapine montre une affinité élevée pour les récepteurs $D_4$ (Van Tol et al., "Cloning of the gene for a human dopamine $D_4$ receptor with high affinity for the antipsychotic clozapine". Nature, 1991 ; 350 : 610-614). En outre, la noradrénaline, un neurotransmetteur impliqué dans les états psychotiques, anxieux et dépressifs ainsi que les perturbations cognitives et attentionnelles (Bloom and Kupfer, 1995 (déjà cité)) possède une affinité élevée pour les récepteurs $D_4$ (Lanau et al., "Epinephrine and norepinephrine act as potent agonists at the human recombinant $D_{4.4}$ receptor". Am. Soc. Neurosci., 1995 ; 21 : 252.2). Ces résultats démontrent l'intérêt des produits de l'invention dans le traitement de la schizophrénie, l'anxiété, la dépression, l'abus des drogues, les états impulsifs (p.e., l'agressivité) et les troubles mnémocognitifs. De plus, la forte concentration des récepteurs $D_4$ dans la couche superficielle de la moëlle épinière (Matsumoto et al., "Low levels of mRNA for dopamine $D_4$ receptor in human cerebral cortex and striatum". J. Neurochem., 1996 ; 66 : 915-919) laisse envisager un intérêt dans le traitement des états douloureux (p.e., neuropathique ou migraineux).

Plus spécifiquement, la présente invention concerne les dérivés de formule générale (I):

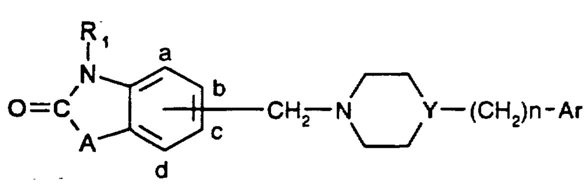

(I)

dans laquelle :

$R_1$ représente un atome d'hydrogène ou un groupement alkyle inférieur, ou encore
$R_1$ représente un groupement

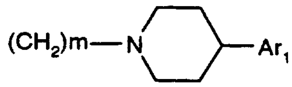

dans laquelle m représente un entier compris inclusivement entre 1 et 4 et $Ar_1$ représente

. ou bien un groupement CO-$Ar_2$ où $Ar_2$ représente un noyau phényle non substitué ou substitué par un ou plusieurs radicaux choisis parmi halogène, hydroxy, alkyle inférieur, trifluorométhyle ou alkoxy inférieur,

. ou bien un groupement =C-$(Ar_2)_2$ où $Ar_2$ a la même signification que précédemment,

n représente 0 ou 1
A représente un atome d'oxygène ou de soufre
Y représente un groupement CH ou un atome d'azote
Ar représente un groupement phényle ou naphtyle éventuellement substitué par un, deux ou trois groupements choisis parmi halogène, hydroxy, alkoxy inférieur, alkyle inférieur, alkoxy inférieur-alkyle inférieur, trifluorométhyle ou aminosulfonyle, ou Ar représente un groupement pyridyle, pyrimidinyle, ou 3-(benzo[d]-1,2-thiazolyle) encore appelé 3-benzisothiazolyle:

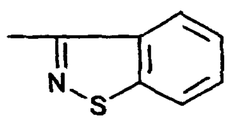

le cas échéant leurs isomères purs ou en mélange ainsi que leurs sels d'addition à un acide pharmaceutiquement acceptable, ou une base pharmaceutiquement acceptable lorsque $R_1$ = H
étant entendu que, sauf indication contraire,

- les termes "alkyle inférieur" et "alkoxy inférieur" correspondent à des groupements linéaires ou ramifiés contenant de 1 à 6 atomes de carbone.

Parmi les acides pharmaceutiquement acceptables on peut citer à titre non limitatif les acides chlorhydrique, sulfurique, tartrique, maléique, fumarique, oxalique, méthane sulfonique, camphorique, éthane sulfonique, citrique.
Parmi les bases pharmaceutiquement acceptables on peut citer à titre non limitatif les hydroxydes de sodium, potassium, calcium ainsi que les carbonates de sodium, potassium, calcium.

L'invention se rapporte préférentiellement aux composés de formule (I) pour lesquels pris ensemble ou séparément :

. $R_1$ est un atome d'hydrogène, un groupement méthyle ou un groupement

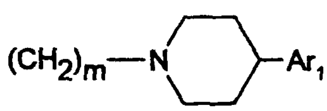

pour lequel m vaut 2 et $Ar_1$ représente ou bien un groupement

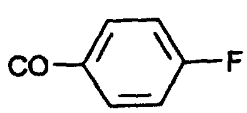

ou encore un groupement

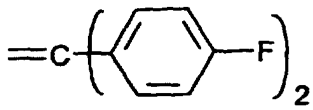

. et A est un atome de soufre ; il s'agit alors de dérivés de la benzothiazolinone de formule :

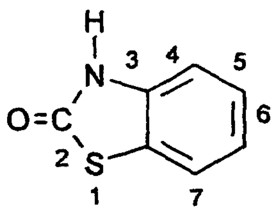

. et A est un atome d'oxygène ; il s'agit alors de dérivés de la benzoxazolinone de formule

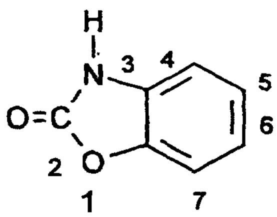

. la chaîne latérale est greffée en position c
. n représente 0
. Y représente un atome d'azote
. Ar représente un groupement phényle substitué par un atome de fluor ou encore par un atome de chlore ou encore par un groupement méthoxy, ainsi que leurs isomères purs ou en mélange ainsi que leurs sels d'addition à un acide pharmaceutiquement acceptable ou une base pharmaceutiquement acceptable lorsque $R_1$ est un atome d'hydrogène.

Préférentiellement l'invention se rapporte à :

. la 3-méthyl-6-{[4-(2-méthoxyphényl)pipérazin-1-yl]méthyl}benzoxazolinone, ainsi que ses sels d'addition à un acide pharmaceutiquement acceptable,

. la 3-méthyl-6-{[4-(2-méthoxyphényl)pipérazin-1-yl]méthyl}benzothiazolinone, ainsi que ses sels d'addition à un acide pharmaceutiquement acceptable,

. la 3-méthyl-6-{[4-(2-fluorophényl)pipérazin-1-yl]méthyl}benzothiazolinone, ainsi que ses sels d'addition à un acide pharmaceutiquement acceptable,

. la 3-méthyl-6-{[4-(2-fluorophényl)pipérazin-1-yl]méthyl}benzoxazolinone, ainsi que ses sels d'addition à un acide pharmaceutiquement acceptable,

. la 3-méthyl-6-{[4-(4-chlorophényl)pipérazin-1-yl]méthyl)benzoxazolinone ainsi que ses sels d'addition à un acide pharmaceutiquement acceptable,

. la 3-méthyl-6-{[4-(4-chlorophényl)pipérazin-1-yl]méthyl}benzothiazolinone ainsi que ses sels d'addition à un acide pharmaceutiquement acceptable,

- la 6-{[4-(2-fluorophényl)pipérazin-1-yl1méthyl}benzoxazolinone ainsi que ses sels d'addition à un acide ou une base pharmaceutiquement acceptables,
- la 6-{[4-(2-fluorophényl)pipérazin-1-yl]méthyl}benzothiazolinone ainsi que ses sels d'addition à un acide ou une base pharmaceutiquement acceptables,
- la 3-{2-[4-(4-fluorobenzoyl)pipéridin-1-yl]éthyl}-6-{[4-(2-fluorophényl)pipérazin-1-yl]méthyl} benzoxazolinone ainsi que ses sels d'addition à un acide pharmaceutiquement acceptable,
- la 3-{2-[4-(4',4"-difluorobenzhydrylidene)pipéridin-1-yl]éthyl}-6-{[4-(2-fluorophényl) pipérazin-1-yl]méthyl}benzoxazolinone ainsi que ses sels d'addition à un acide pharmaceutiquement acceptable,
- la 3-{2-[4-(4-fluorobenzoyl)pipéridin-1-yl]éthyl}-6-{[4-(2-fluorophényl)pipérazin-1-yl]méthyl} benzothiazolinone ainsi que ses sels d'addition à un acide pharmaceutiquement acceptable,
- la 3-méthyl-6-{[4-(benzo[d]1,2-thiazolyl)pipérazin-1-yl]méthyl}benzoxazolinone, ainsi que ses sels d'addition à un acide pharmaceutiquement acceptable,
- la 3-méthyl-6-{[4-(benzo[d]1,2-thiazolyl)pipérazin-1-yl]méthyl}benzothiazolinone, ainsi que ses sels d'addition à un acide pharmaceutiquement acceptable,
- la 3-méthyl-6-{[4-(4-aminosulfonylphényl)pipérazin-1-yl]méthyl}benzoxazolinone ainsi que ses sels d'addition à un acide pharmaceutiquement acceptable.
- la 3-méthyl-6-{[4-(4-aminosulfonylphényl)pipérazin-1-yl]méthyl}benzothiazolinone ainsi que ses sels d'addition à un acide pharmaceutiquement acceptable,
- la 3-méthyl-6-{[4-(3-méthoxyphényl)pipérazin-1-yl]méthyl}benzoxazolinone ainsi que ses sels d'addition à un acide pharmaceutiquement acceptable,
- la 3-méthyl-6-{[4-(4-méthoxyphényl)pipérazin-1-yl]méthyl}benzothiazolinone ainsi que ses sels d'addition à un acide pharmaceutiquement acceptable,
- la 3-méthyl-6-{[4-(4-méthoxyphényl)pipérazin-1-yl]méthyl)benzoxazolinone ainsi que ses sels d'addition à un acide pharmaceutiquement acceptable,

L'invention s'étend également au procédé de préparation des composés de formule (I), caractérisé

- d'une part, lorsque dans le composé de formule (I) que l'on veut obtenir le groupement $R_1$ représente un groupement alkyle inférieur, en ce que l'on fait réagir un composé de formule (II) :

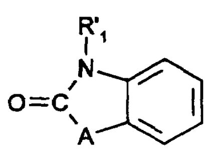

(II)

dans laquelle A a la même signification que précédemment et $R'_1$ représente un groupement alkyle inférieur avec l'hexaméthylène tétramine, préférentiellement en milieu acide pour conduire à un produit de formule (III) :

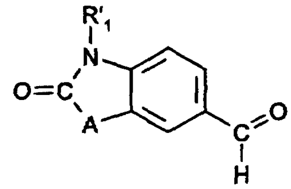

(III)

où $R'_1$ et A ont la même signification que précédemment que l'on traite par un agent d'hydrogénation pour conduire à un produit de formule (IV):

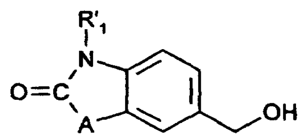

(IV)

où $R'_1$ et A ont la même signification que précédemment,

. d'autre part, lorsque dans le composé de formule (I) que l'on souhaite obtenir $R_1$ est différent du groupement alkyle inférieur en ce que l'on traite un dérivé de formule (V):

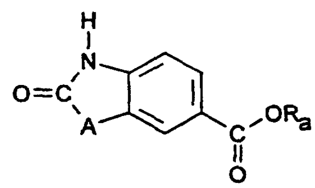

(V)

où $R_a$ représente un groupement alkyle inférieur et A a la même signification que précédemment, par un agent d'hydrogénation pour obtenir un dérivé de formule (IVb):

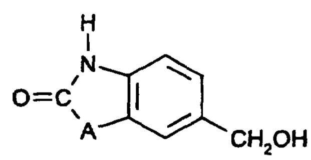

(IVb)

où A a la même signification que précédemment

. dérivé de formule (IV) ou (IVb) que l'on traite par un agent d'halogénation pour conduire à un produit de formule (VI):

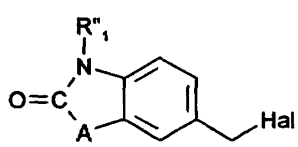

(VI)

où R"$_1$ représente un atome d'hydrogène ou un groupement alkyle inférieur et A a la même signification que précédemment et Hal représente un atome d'halogène
que l'on traite par une amine de formule (VII) :

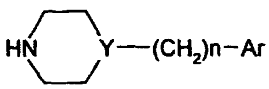

(VII)

où Y, n et Ar ont la même signification que dans la formule (I)
pour obtenir un produit de formule (I/a)

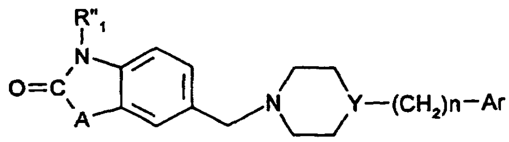

(I/a)

cas particulier des dérivés de formule (1), R"$_1$, A, Y, n et Ar ayant la même signification que précédemment
dérivé de formule I/a, qui, lorsque dans le dérivé de formule (I) que l'on souhaite obtenir R$_1$ ne représente ni un atome d'hydrogène ni un groupement alkyle inférieur, est traité par un dérivé de formule (IX) :

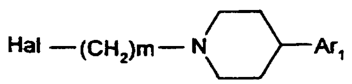

(IX)

où Hal représente un atome d'halogène, et m et Ar$_1$ ont la même définition que dans la formule (I)
pour conduire à un dérivé de formule I/c :

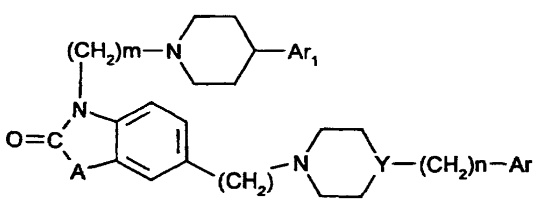

(I/c)

cas particulier des dérivés de formule (I) pour lesquels R$_1$ représente un groupement

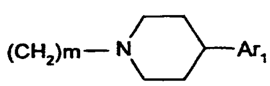

le dérivé de formule I/a ou I/c ainsi obtenu étant le cas échéant

- purifié par une ou plusieurs méthodes choisies parmi cristallisation, chromatographie, extraction, passage sur résine ou charbon
- séparé le cas échéant, sous forme pure ou sous forme de mélange, en ses isomères optiques,
- et si on le désire salifié par un acide pharmaceutiquement acceptable, ou une base pharmaceutiquement acceptable.

Les composés de formule III, IV, IVb et VI sont nouveaux et à ce titre font partie de l'invention au même titre que les dérivés de formule (I) dont ils constituent des intermédiaires de synthèse à l'exception du dérivé de formule III pour lequel A représente un atome d'oxygène et $R'_1$ un groupement méthyle (Renard et coll.; Bull. Soc. Pharm. Lille, 1979, 2-3, 125-138). Plus particulièrement parmi ces dérivés on préfère :

. la 3-méthyl-6-formyl benzothiazolinone,

. la 3-méthyl-6-hydroxyméthyl benzoxazolinone,

. la 3-méthyl-6-hydroxyméthyl benzothiazolinone,

. la 6-hydroxyméthyl benzoxazolinone,

. la 6-hydroxyméthyl benzothiazolinone,

. les 3-méthyl-6-halogénométhyl benzoxazolinones,

. les 3-méthyl-6-halogénométhyl benzothiazolinones,

. les 6-halogénométhyl benzoxazolinones,

. les 6-halogénométhyl benzothiazolinones.

Les composés de formule (I) possèdent des propriétés pharmacologiques intéressantes.

Les essais de binding ont montré que les composés de l'invention se comportent comme de très puissants ligands des récepteurs $D_4$. Cette affinité est accompagnée d'une très grande sélectivité vis à vis des autres récepteurs notamment $D_2$. Ceci est d'autant plus surprenant que les composés de l'art antérieur précédemment cités n'ont pas d'affinité pour le récepteur $D_4$ mais possèdent par contre une haute affinité pour les récepteurs $D_2$.

Les composés de l'invention sont peu toxiques et de par leur profil réceptoriel devraient posséder une bonne activité dans la schizophrénie et dans diverses catégories de psychoses, dans la modulation de l'humeur et des fonctions cognitives, dans l'anxiété, la dépression, l'abus des drogues, les états impulsifs et les troubles mnémocognitifs ainsi que les états migraineux.

La présente invention a également pour objet les compositions pharmaceutiques renfermant comme principe actif au moins un composé de formule générale (I) ou un de ses sels d'addition à un acide pharmaceutiquement acceptable ou le cas échéant à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublingaux, les sachets, paquets, les gélules, glossettes, tablettes, suppositoires, crèmes, pommades, gels dermiques etc...

La posologie varie selon l'âge et le poids du patient, la nature et la sévérité de l'affection ainsi que la voie d'administration. Celle-ci peut être orale, nasale, rectale ou parentérale.

D'une manière générale, la posologie unitaire s'échelonne entre 0,05 mg et 30 mg pour les affections du comportement psychique de une à trois prises par 24 heures.

Les exemples suivants illustrent l'invention et ne la limitent en aucune façon.

Les spectres de résonance magnétique nucléaire $^1H$ ont été réalisés en utilisant le TMS (tétraméthylsilane) comme référence interne. Les déplacements chimiques sont exprimés en partie par million (p.p.m.). Les spectres infrarouge ont été effectués sous forme de pastille de bromure de potassium renfermant environ 1 % du produit à analyser.

Les préparations sauf indication contraire ne font pas partie de l'invention mais sont utiles pour réaliser la synthèse des dérivés de l'invention.

**Préparation 1 : 3-METHYLBENZOTHIAZOLINONE**

Dans 2000 $cm^3$ d'eau, dissoudre 1 mole d'hydroxyde de sodium, ajouter 1 mole de benzothiazolinone puis, goutte à goutte, 1 mole de sulfate de méthyle. Agiter pendant 3 heures sous agitation magnétique, à température

ambiante. Essorer, laver à l'eau, sécher et recristalliser le produit dans le propan-2-ol.

Masse molaire : 165,21 g.mol$^{-1}$ pour $C_8H_7NOS$
Point de fusion : 74°C
Rendement : 76 %
Rf : 0,7 Eluant : cyclohexane/toluène/acétone (5/2/3)

Spectrométrie dans l'infrarouge

| | |
|---|---|
| 2940-2910 cm$^{-1}$ | νCH |
| 1680 cm$^{-1}$ | νCO-S |
| 1580 cm$^{-1}$ | νC=C |

Spectrométrie de résonance magnétique nucléaire (80 MHz, DMSO/d6)

| | | | |
|---|---|---|---|
| δ : 3,45 ppm | singulet | 3H | N-$CH_3$ |
| δ : 7,30 ppm | massif | 4 H | aromatiques |

**<u>Préparation 2</u> : 3-METHYLBENZOXAZOLINONE**

Dans 2000 cm$^3$ d'eau, sous agitation magnétique, dissoudre 1 mole d'hydroxyde de sodium, 1 mole de benzoxazolinone puis ajouter goutte à goutte 1 mole de sulfate de méthyle. Poursuivre l'agitation pendant 3 heures. Filtrer le précipité obtenu, le laver avec une solution aqueuse (2 fois 200 cm$^3$) d'hydroxyde de sodium à 1 % puis à l'eau jusqu'à neutralité des eaux de lavage. Sécher et recristalliser le produit dans l'alcool à 95°.

Masse molaire : 149,15 g.mol$^{-1}$ pour $C_8H_7NO_2$
Point de fusion : 86 °C
Rendement : 76 %
Rf : 0,9 Eluant : méthanol (saturé d'ammoniac) / chloroforme (1/9)

Spectrométrie dans l'infrarouge

| | |
|---|---|
| 3050 cm$^{-1}$ | νCH |
| 1760 cm$^{-1}$ | νCO-O |
| 1590 cm$^{-1}$ | νC=C |

Spectrométrie de résonance magnétique nucléaire (80 MHz, DMSO/d6)

| | | | |
|---|---|---|---|
| δ:3,35 ppm | singulet | 3H | N-$CH_3$ |
| δ:7,40 ppm | massif | 4H | aromatiques |

**<u>Préparation 3</u> : 3-METHYL-6-FORMYLBENZOTHIAZOLINONE**

Ecraser au mortier 0,010 mole de 3-méthylbenzothiazolinone et 0,015 mole d'hexaméthylène tétramine puis introduire successivement les deux produits dans un ballon contenant 50 g d'acide polyphosphorique à 130°C, sous agitation mécanique dans un bain d'huile. Chauffer à 130°C pendant 20 minutes. Après refroidissement, le mélange réactionnel est versé dans 300 cm$^3$ d'eau glacée. Agiter pendant 1 heure. Essorer, laver à l'eau et sécher le précipité. Extraire le filtrat à 3 reprises par 30 cm$^3$ de chloroforme. Les phases chloroformiques sont lavées à l'eau, séchées sur

chlorure de calcium et évaporées sous pression réduite. Sécher et recristalliser le produit dans l'éthanol absolu.

Masse molaire : 193,22 g.mol$^{-1}$ pour $C_9H_7NO_2S$
Point de fusion : 135°C
Rendement : 73 %
Rf : 0,8 Eluant : cyclohexane/toluènelacétone (5/2/3)

Spectrométrie dans l'infrarouge

| | |
|---|---|
| 1680 cm$^{-1}$<br>1590 cm$^{-1}$ | νCO-S et νCO<br>νC=C |

Spectrométrie de résonance magnétique nucléaire (80 MHz, DMSO/d6)

| | | | | |
|---|---|---|---|---|
| δ : 3,45 ppm | singulet | 3H | N-$CH_3$ | |
| δ : 7,50 ppm | doublet | 1H | $H_4$ | $J_{4-5}$ = 8,30 Hz |
| δ : 7,80 ppm | doublet dédoublé | 1 H | $H_5$ | $J_{5-4}$ = 8,30 Hz $J_{5-7}$ = 1,30 Hz |
| δ : 8,20 ppm | doublet | 1 H | $H_7$ | $J_{7-5}$ = 1,30 Hz |
| δ : 9,90 ppm | singulet | 1 H | CHO | |

Remarque : Ce composé (préparation 3) fait partie de l'invention au même titre que les dérivés de formule (I).

**Préparation 4 : 3-METHYL-6-FORMYLBENZOXAZOLINONE**

Mélanger dans un mortier 0,10 mole de 3-méthylbenzoxazolinone et 0,15 mole d'hexaméthylènetétramine ; introduire le mélange dans un ballon contenant 200 g d'acide polyphosphorique à 90°C, sous agitation dans un bain d'huile. Chauffer à 150°C pendant 10 minutes en agitant. Après refroidissement le mélange réactionnel est versé dans 500 cm$^3$ d'eau glacée et agité pendant une heure. Essorer, laver à l'eau et sécher le précipité obtenu. Le filtrat est soumis à plusieurs extractions au chloroforme. Les phases chloroformiques réunies sont lavées à l'eau, séchées sur du chlorure de calcium et évaporées sous pression réduite. Le produit obtenu, joint au précipité précédent est recristallisé dans l'eau.

Masse molaire : 177,16 g mol$^{-1}$ pour $C_9H_7NO_3$
Point de fusion : 146°C
Rendement : 74 %
Rf : 0,8 Eluant : méthanol (saturé d'ammoniac)/chloroforme (1/9)

Spectrométrie dans l'infrarouge

| | |
|---|---|
| 3040 cm$^{-1}$<br>1765 cm$^{-1}$<br>1675 cm$^{-1}$<br>1600 cm$^{-1}$ | νCH<br>νCO-O<br>νCO<br>νC=C |

Spectrométrie de résonance magnétique nucléaire (300 MHz, DMSO/d6)

| | | | | |
|---|---|---|---|---|
| δ : 3,40 ppm | singulet | 3H | N-$CH_3$ | |

(suite)

| | | | | |
|---|---|---|---|---|
| δ : 7,50 ppm | doublet | 1H | $H_4$ | $J_{4-5}$ = 8,30 Hz |
| δ : 7,80 ppm | doublet dédoublé | 1H | $H_5$ | $J_{5-4}$ = 8,30 Hz $J_{5-7}$ = 1,30 Hz |
| δ: 8,00 ppm | doublet | 1 H | $H_7$ | $J_{7-5}$ = 1,30 Hz |
| δ : 9,80 ppm | singulet | 1H | CHO | |

**<u>Préparation 5</u> : 3-METHYL-6-HYDROXYMETHYLBENZOTHIAZOLINONE**

Dans 50 $cm^3$ de méthanol, introduire 0,010 mole de 3-méthyl-6-formyl benzothiazolinone à température ambiante. Ajouter 0,015 mole de borohydrure de sodium par petites fractions sous agitation magnétique. Après 2 heures d'agitation à température ambiante, le méthanol est évaporé sous pression réduite puis le résidu est repris par 50 $cm^3$ d'eau. Le précipité est essoré, séché puis recristallisé dans le propan-2-ol.

Masse molaire : 195,24 $g.mol^{-1}$ pour $C_9H_9NO_2S$
Point de fusion : 120°C
Rendement : 79 %
Rf : 0,5 Eluant : cyclohexane/toluène/acétone (5/2/3)

Spectrométrie dans l'infrarouge

| | |
|---|---|
| 3350 $cm^{-1}$ | νOH |
| 2960-2800 $cm^{-1}$ | νCH |
| 1670 $cm^{-1}$ | νCO-S |
| 1600 $cm^{-1}$ | νC=C |

Spectrométrie de résonance magnétique nucléaire (80 MHz, DMSO/d6)

| | | | | |
|---|---|---|---|---|
| δ : 3,40 ppm | singulet | 3H | $N-CH_3$ | |
| δ : 4,50 ppm | singulet | 2H | $CH_2$ | |
| δ : 5,25 ppm | signal | 1H | OH | échangeable avec $D_2O$ |
| δ : 7,30 ppm | massif | 2H | $H_4$, $H_5$ | |
| δ : 7,60 ppm | singulet | 1H | $H_7$ | |

Remarque : Ce composé (préparation 5) fait partie de l'invention au même titre que les dérivés de formule (I).

**<u>Préparation 6</u> : 3-METHYL-6-HYDROXYMETHYLBENZOXAZOLINONE**

Dans 150 $cm^3$ de méthanol, introduire 0,10 mole de 3-méthyl-6-formyl benzoxazolinone. Ajouter, à température ambiante, 0,15 mole de borohydrure de sodium par petites fractions sous agitation magnétique. Après 2 heures le méthanol est évaporé sous pression réduite et le résidu repris par de l'eau. Essorer puis recristalliser le précipité dans l'eau.

Masse molaire : 179,17 $g.mol^{-1}$ pour $C_9H_9NO_3$
Point de fusion : 127°C
Rendement : 73 %
Rf : 0,5 Eluant : méthanol (saturé d'ammoniac)/chloroforme (1/9)

Spectrométrie dans l'infrarouge

| | |
|---|---|
| 3500-3200 $cm^{-1}$ | νOH |
| 3080-2880 $cm^{-1}$ | νCH |
| 1755 $cm^{-1}$ | νCO-O |
| 1620 $cm^{-1}$ | νC=C |

Spectrométrie de résonance magnétique nucléaire (80 MHz, DMSO/d6)

| | | | | |
|---|---|---|---|---|
| δ : 3,35 ppm | singulet | 3H | N-$CH_3$ | |
| δ : 4,55 ppm | singulet | 2H | $CH_2$ | |
| δ : 5,25 ppm | signal | 1H | OH | échangeable avec $D_2O$ |
| δ : 7,20 ppm | massif | 2 H | $H_4$, $H_5$ | |
| δ : 7,25 ppm | singulet | 1H | $H_7$ | |

Remarque : Ce composé (préparation 6) fait partie de l'invention au même titre que les dérivés de formule (I).

**Préparation 7 : 6-HYDROXYMETHYLBENZOXAZOLINONE**

Dans 50 $cm^3$ de tétrahydrofuranne, refroidi dans un bain de glace, introduire 0,01 mole de 6-éthoxycarbonylbenzoxazolinone et 0,03 mole d'hydrure de lithium aluminium par petites fractions. Agiter à température ambiante pendant 1 heure. Hydrolyser dans 100 $cm^3$ d'eau glacée acidifiée à pH = 1 avec de l'acide chlorhydrique 6N. Extraire par 3 fois 50 $cm^3$ de chloroforme, sécher puis évaporer la phase organique. Sécher et recristalliser le composé dans l'acétonitrile.

Masse molaire : 165,14 g.$mol^{-1}$ pour $C_8H_7NO_3$
Point de fusion : 153°-154°C
Rendement : 48 %
Rf : 0,2 Eluant : méthanol (saturé d'ammoniac)/chloroforme (1/9)

Spectrométrie dans l'infrarouge

| | |
|---|---|
| 3330 $cm^{-1}$ | νOH et νNH |
| 3100-2700 $cm^{-1}$ | νCH |
| 1740 $cm^{-1}$ | νCO-O |
| 1620 $cm^{-1}$ | νC=C |

Spectrométrie de résonance magnétique nucléaire (80 MHz, DMSO/d6)

| | | | |
|---|---|---|---|
| δ: 4,60 ppm | singulet | 2H | $CH_2$ |
| δ: 5,30 ppm | signal | 1H | OH échangeable avec $D_2O$ |
| δ: 7,00 ppm | massif | 2H | $H_4$, $H_5$ |
| δ: 7,30 ppm | singulet | 1H | $H_7$ |
| δ: 11,60 ppm | singulet | 1H | NH échangeable avec $D_2O$ |

Remarque : Ce composé (préparation 7) fait partie l'invention au même titre que les dérivés de formule (I).

**<u>Préparation 8</u> : 3-METHYL-6-CHLOROMETHYLBENZOTHIAZOLINONE**

Dans 50 $cm^3$ de chloroforme, introduire 0,01 mole de 3-méthyl-6-hydroxyméthyl benzothiazolinone. Ajouter goutte à goutte, à l'aide d'une ampoule à addition, 0,02 mole de chlorure de thionyle. Le mélange est porté à reflux pendant 4 heures. Le chloroforme est évaporé sous pression réduite puis le résidu est repris 3 fois par de l'éthanol absolu pour entraîner les traces de chlorure de thionyle. Sécher et recristalliser le produit dans le propan-2-ol.

Masse molaire : 213,68 $g.mol^{-1}$ pour $C_9H_8ClNOS$
Point de fusion : 128°C
Rendement : 95 %
Rf : 0,8 Eluant : cyclohexane/toluène/acétonè (5/2/3)

Spectrométrie dans l'infrarouge

| | |
|---|---|
| 1680 $cm^{-1}$ | νCO-S |
| 1600 $cm^{-1}$ | νC=C |

Spectrométrie de résonance magnétique nucléaire (300 MHz, DMSO/d6)

| | | | | |
|---|---|---|---|---|
| δ : 3,40 ppm | singulet | 3H | $N-CH_3$ | |
| δ : 4,80 ppm | singulet | 2H | $CH_2$ | |
| δ : 7,25 ppm | doublet | 1H | $H_4$ | $J_{4-5}$ = 8,30 Hz |
| δ : 7,45 ppm | doublet dédoublé | 1H | $H_5$ | $J_{5-4}$ = 8,30 Hz $J_{5-7}$ = 1,30 Hz |
| δ : 7,75 ppm | doublet | 1H | $H_7$ | $J_{7-5}$ = 1,30 Hz |

Remarque : Ce composé (préparation 8) fait partie de l'invention au même titre que les dérivés de formule (I).

**<u>Préparation 9</u> : 3-METHYL-6-CHLOROMETHYLBENZOXAZOLINONE**

Dans 100 $cm^3$ de chloroforme, dissoudre 0,10 mole de 3-méthyl-6-hydroxyméthyl benzoxazolinone. Ajouter goutte à goutte, à l'aide d'une ampoule à addition, 0,20 mole de chlorure de thionyle. Le mélange est porté à reflux pendant 4 heures. Le chloroforme est évaporé sous pression réduite puis le résidu est repris 3 fois par de l'éthanol absolu pour entraîner les traces de chlorure de thionyle. Sécher puis recristalliser le produit dans le propanol-2-ol.

Masse molaire : 197,62 $g.mol^{-1}$ pour $C_9H_8ClNO_2$
Point de fusion : 132,5°C
Rendement : 84 %
Rf : 0,8 Eluant : méthanol (saturé d'ammoniac)/chloroforme (1/9)

Spectrométrie dans l'infrarouge

| | |
|---|---|
| 3040 $cm^{-1}$ | νCH |
| 1755 $cm^{-1}$ | νCO-O |
| 1610 $cm^{-1}$ | νC=C |

Spectrométrie de résonance magnétique nucléaire (80 MHz, DMSO/d6)

| | | | |
|---|---|---|---|
| δ : 3,35 ppm | singulet | 3H | N-$CH_3$ |
| δ : 4,80 ppm | singulet | 2H | $CH_2$ |
| δ : 7,30 ppm | massif | 2H | $H_4$, $H_5$ |
| δ : 7,45 ppm | singulet | 1H | $H_7$ |

Remarque : Ce composé (préparation 9) fait partie de l'invention au même titre que les dérivés de formule (I).

**Préparation 10 : 6-CHLOROMETHYLBENZOXAZOLINONE**

Dans 50 $cm^3$ de choroforme, introduire 0,01 mole de 6-hydroxyméthyl benzoxazolinone. Ajouter goutte à goutte, à l'aide d'une ampoule à addition, 0,02 mole de chlorure de thionyle. Le mélange est porté à reflux pendant 2 heures. Le chloroforme est évaporé sous pression réduite, puis le résidu est repris 3 fois par de l'éthanol absolu pour entraîner les traces de chlorure de thionyle. Sécher et recristalliser le produit dans le propan-2-ol.

Masse molaire : 183,59 $g.mol^{-1}$ pour $C_8H_6ClNO_2$
Point de fusion : 186-187°C
Rendement : 72 %
Rf : 0,5 Eluant : méthanol (saturé d'ammoniac)/chloroforme (1/9)

Spectrométrie dans l'infrarouge

| | |
|---|---|
| 3200 $cm^{-1}$ | νNH |
| 1760 $cm^{-1}$ | νCO-O |
| 1610 $cm^{-1}$ | νC=C |

Spectrométrie de résonance magnétique nucléaire (80 MHz, DMSO/d6)

| | | | |
|---|---|---|---|
| δ : 4,80 ppm | singulet | 2H | $CH_2$ |
| δ : 7,20 ppm | massif | 3H | $H_4$, $H_5$, $H_7$ |
| δ : 11,75 ppm | singulet | 1H | NH échangeable avec $D_2O$ |

Remarque : Ce composé (préparation 10) fait partie de l'invention au même titre que les dérivés de formule (I).

**Préparation 11 : 6-HYDROXYMETHYLBENZOTHIAZOLINONE**

Dans 50 $cm^3$ de tétrahydrofuranne, refroidi dans un bain de glace, introduire 0,01 mole de 6-éthoxycarbonylbenzothiazolinone et 0,03 mole d'hydrure de lithium aluminium par petites fractions. Agiter à température ambiante pendant 30 minutes. Hydrolyser dans 100 $cm^3$ d'eau glacée acidifiée à pH = 1 avec de l'acide chlorhydrique 6N. Extraire par 3 fois 50 $cm^3$ de chloroforme, sécher puis évaporer la phase organique. Sécher et recristalliser le composé dans l'acétonitrile.

Masse molaire : 181,21 $g.mol^{-1}$ pour $C_8H_7NO_2S$
Point de fusion : 168-170°C
Rendement : 40 %
Rf : 0,2 Eluant : méthanol (saturé d'ammoniac)/chloroforme (1/9)

Spectrométrie dans l'infrarouge

| | |
|---|---|
| 3300 $cm^{-1}$ | νOH et νNH |

(suite)

| | |
|---|---|
| 3000-2800 $cm^{-1}$<br>1650 $cm^{-1}$ | νCH<br>νCO-S |

Spectrométrie de résonance magnétique nucléaire (300 MHz, DMSO/d6)

| | | | | |
|---|---|---|---|---|
| δ: 4,50 ppm | singulet | 2H | $CH_2$ | |
| δ: 5,20 ppm | signal | 1H | OH | échangeable avec $D_2O$ |
| δ; 7,10 ppm | doublet | 1H | $H_4$ | $J_{4-5}$ = 8,00 Hz |
| δ: 7,25 ppm | doublet | 1H | $H_5$ | $J_{5-4}$ = 8,00 Hz |
| δ: 7,50 ppm | singulet | 1H | $H_7$ | |
| δ: 11,85 ppm | singulet | 1 H | NH | échangeable avec $D_2O$ |

Remarque : Ce composé (préparation 11) fait partie de l'invention au même titre que les dérivés de formule (I).

**<u>Préparation 12</u> : 6-CHLOROMETHYLBENZOTHIAZOLINONE**

Dans 50 $cm^3$ de chloroforme, introduire 0,01 mole de 6-hydroxyméthyl benzothiazolinone et ajouter goutte à goutte, à l'aide d'une ampoule à addition, 0,02 mole de chlorure de thionyle. Le mélange est porté à reflux pendant 2 heures. Le chloroforme est évaporé sous pression réduite, puis le résidu est repris 3 fois par de l'éthanol absolu pour entraîner les traces de chlorure de thionyle. Sécher et recristalliser le produit dans le toluène.

Masse molaire : 199,66 g.$mol^{-1}$ pour $C_8H_6ClNOS$
Point de fusion : 183-184°C
Rendement : 73 %
Rf : 0,6    Eluant : méthanol (saturé d'ammoniac)/chloroforme (1/9)

Spectrométrie dans l'infrarouge

| | |
|---|---|
| 3140 $cm^{-1}$ | νNH |
| 3080-2820 $cm^{-1}$ | νCH |
| 1660 $cm^{-1}$ | νCO-S |
| 1600 $cm^{-1}$ | νC=C |

Spectrométrie de résonance magnétique nucléaire (300 MHz, DMSO/d6)

| | | | | |
|---|---|---|---|---|
| δ : 4,80 ppm | singulet | 2H | $CH_2$ | |
| δ : 7,10 ppm | doublet | 1H | $H_4$ | $J_{4-5}$ = 8,25 Hz |
| δ : 7,50 ppm | doublet | 1H | $H_5$ | $J_{5-4}$ = 8,25 Hz |
| δ : 7,65 ppm | singulet | 1H | $H_7$ | |
| δ : 12,00 ppm | singulet | 1H | NH | échangeable avec $D_2O$ |

Remarque : Ce composé (préparation 12) fait partie de l'invention au même titre que les dérivés de formule (I).

**<u>EXEMPLE 1</u> : CHLORHYDRATE DE LA 3-METHYL-6-[(4-(2-METHOXYPHENYL) PIPERAZIN-1-YL)METHYL] BENZOTHIAZOLINONE**

Dans 20 $cm^3$ de dioxanne, ajouter 0,01 mole de 3-méthyl-6-chlorométhyl benzothiazolinone, 0,012 mole du

chlorhydrate de la N-orthométhoxyphénylpipérazine et 0,022 mole de triéthylamine. Chauffer à reflux, sous agitation magnétique, pendant 6 jours. Filtrer le résidu minéral puis évaporer sous pression réduite de dioxanne. Reprendre par 50 $cm^3$ d'eau le résidu puis le filtrer et le sécher. Dissoudre le produit dans le minimum d'éthanol absolu, ajouter 100 $cm^3$ d'éthanol absolu saturé d'acide chlorhydrique gazeux. Filtrer, laver le précipité à l'éthanol absolu, le sécher puis le recristalliser dans le méthanol.

Masse molaire : 405,95 $g.mol^{-1}$ pour $C_{20}H_{24}ClN_3O_2S$
Point de fusion : 168°C
Rendement : 33 %
Rf : 0,8 Eluant : cyclohexane/toluène/acétone (5/2/3)

Spectrométrie dans l'infrarouge

| | |
|---|---|
| 3060-2840 $cm^{-1}$ | νCH |
| 2640-2060 $cm^{-1}$ | $νNH^+$ |
| 1670 $cm^{-1}$ | νCO-S |
| 1600 $cm^{-1}$ | νC=C |

Spectrométrie de résonance magnétique nucléaire (300 MHz, DMSO/d6)

| | | | | |
|---|---|---|---|---|
| δ: 3,00 ppm | massif | 4H | pipérazine | |
| δ: 3,40 ppm | massif | 4H | pipérazine | |
| δ: 3,45 ppm | singulet | 3H | $N-CH_3$ | |
| δ: 3,80 ppm | singulet | 3H | $O-CH_3$ | |
| δ: 4,40 ppm | singulet | 2H | $CH_2$ | |
| δ: 7,00 ppm | massif | 4H | phényl | |
| δ: 7,45 ppm | doublet | 1H | $H_4$ | $J_{4-5}$ = 8,30 Hz |
| δ: 7,65 ppm | doublet | 1H | $H_5$ | $J_{5-4}$=8,30 Hz |
| δ: 7,90 ppm | singulet | 1H | $H_7$ | |
| δ: 11,00 ppm | signal | 1H | $NH^+$ | échangeable avec $D_2O$ |

**<u>EXEMPLE 2</u> : CHLORHYDRATE DE LA 3-METHYL-6-[(4-(2-METHOXYPHENYL) PIPERAZIN-1-YL)METHYL]BENZOXAZOLINONE**

Dans 50 $cm^3$ d'acétone, ajouter 0,010 mole de 3-méthyl-6-chlorométhyl benzoxazolinone, 0,020 mole de triéthylamine, 0,012 mole de N-orthométhoxyphényl pipérazine et 0,001 mole d'iodure de potassium. Chauffer à reflux, sous agitation magnétique, pendant 4 jours. Filtrer le résidu minéral puis évaporer le filtrat à sec sous pression réduite. Ajouter 20 $cm^3$ d'acide chlorhydrique 1N et 30 $cm^3$ d'acétate d'éthyle. Laisser sous agitation pendant 30 minutes. Filtrer le précipité obtenu, le laver à plusieurs reprises avec de l'acétate d'éthyle, le sécher puis le recristalliser dans le méthanol.

Masse molaire : 389,88 $g.mol^{-1}$ pour $C_{20}H_{24}ClN_3O_3$
Point de fusion : > 260°C (forme chlorhydrate)
Point de fusion : 149-150°C (forme base)
Rendement : 52 %
Rf : 0,8 Eluant : méthanol (saturé d'ammoniac)/chloroforme (1/9)

Spectrométrie dans l'infrarouge

| | |
|---|---|
| 3060-2820 $cm^{-1}$ | νCH |
| 2700-2300 $cm^{-1}$ | $νNH^+$ |
| 1780 $cm^{-1}$ | νCO-O |
| 1610 $cm^{-1}$ | νC=C |
| 1590 $cm^{-1}$ | νC=C |

Spectrométrie de résonance magnétique nucléaire (300 MHz, DMSO/d6)

| | | | | |
|---|---|---|---|---|
| δ : 3,05 ppm | massif | 4H | pipérazine | |
| δ : 3,40 ppm | massif | 7H | $N-CH_3$, pipérazine | |
| δ : 3,80 ppm | singulet | 3H | $O-CH_3$ | |
| δ : 4,40 ppm | singulet | 2H | $CH_2$ | |
| δ : 6,95 ppm | massif | 4H | phényl | |
| δ : 7,40 ppm | doublet | 1H | H4 | $J_{4-5}$ = 8,00 Hz |
| δ : 7,50 ppm | doublet | 1H | $H_5$ | $J_{5-4}$ = 8,00 Hz |
| δ : 7,65 ppm | singulet | 1H | $H_7$ | |
| δ : 11,45 ppm | signal | 1H | $NH^+$ | échangeable avec $D_2O$ |

**<u>EXEMPLE 3</u> : 3-METHYL-6-[(4-(2-FLUOROPHENYL)PIPERAZIN-1-YL)METHYL] BENZOTHIAZOLINONE**

Dans 20 $cm^3$ de dioxanne, ajouter 0,01 mole de 3-méthyl-6-chlorométhyl benzothiazolinone, 0,01 mole de N-orthofluorophénylpipérazine et 0,01 mole de triéthylamine. Chauffer à reflux, sous agitation magnétique, pendant 3 jours. Filtrer le résidu minéral puis évaporer sous pression réduite de dioxanne. Reprendre par 50 $cm^3$ d'eau le résidu puis le filtrer, le sécher et le recristalliser dans le propan-1-ol.

Masse molaire : 357,45 $g.mol^{-1}$ pour $C_{19}H_{20}FN_3OS$
Point de fusion : 152°C
Rendement : 60 %
Rf : 0,8 Eluant : cyclohexane/toluène/acétone (5/2/3)

Spectrométrie dans l'infrarouge

| | |
|---|---|
| 2960-2760 $cm^{-1}$ | νCH |
| 1670 $cm^{-1}$ | νCO-S |
| 1600 $cm^{-1}$ | νC=C |

Spectrométrie de résonance magnétique nucléaire (300 MHz, DMSO/d6)

| | | | | |
|---|---|---|---|---|
| δ : 3,00 ppm | massif | 4H | pipérazine | |
| δ : 3,35 ppm | massif | 4H | pipérazine | |
| δ : 3,40 ppm | singulet | 3H | $N-CH_3$ | |
| δ : 3,55 ppm | singulet | 2H | $CH_2$ | |
| δ : 7,00 ppm | massif | 4H | phényl | |
| δ : 7,25 ppm | doublet | 1H | $H_4$ | $J_{4-5}$ = 8,25 Hz |

(suite)

| | | | | |
|---|---|---|---|---|
| δ : 7,35 ppm | doublet | 1H | $H_5$ | $J_{5-4}$ = 8,25 Hz |
| δ : 7,65 ppm | singulet | 1H | $H_7$ | |

**EXEMPLE 4 : CHLORHYDRATE DE LA 3-METHYL-6-[(4-(2-FLUOROPHENYL) PIPERAZIN-1-YL)METHYL]BENZOXAZOLINONE**

Il est identique à celui décrit pour l'obtention de l'exemple 2 en remplaçant la N-orthométhoxyphénylpipérazine par la N-orthofluorophénylpipérazine.

Temps de réaction : 3 jours
Masse molaire : 377,84 g.mol$^{-1}$ pour $C_{19}H_{21}ClFN_3O_2$
Point de fusion : > 260°C (forme chlorhydrate)
Point de fusion : 134-135°C (forme base)
Rendement : 50 %
Solvant de recristallisation : méthanol
Rf : 0,8 Eluant : méthanol (saturé d'ammoniac)/chloroforme (1/9)

Spectrométrie dans l'infrarouge

| | |
|---|---|
| 3040-2820 cm$^{-1}$ | νCH |
| 2740-2320 cm$^{-1}$ | $\nu NH^+$ |
| 1770 cm$^{-1}$ | νCO-O |
| 1610 cm$^{-1}$ | νC=C |

Spectrométrie de résonance magnétique nucléaire (300 MHz, DMSO/d6)

| | | | | |
|---|---|---|---|---|
| δ : 3,15 ppm | massif | 4H | pipérazine | |
| δ : 3,20 ppm | singulet | 3H | $N-CH_3$ | |
| δ : 3,50 ppm | massif | 4H | pipérazine | |
| δ : 4,40 ppm | singulet | 2H | $CH_2$ | |
| δ : 7,10 ppm | massif | 4H | phényl | |
| δ : 7,40 ppm | multiplet | 1H | $H_4$ | |
| δ : 7,45 ppm | multiplet | 1H | $H_5$ | |
| δ : 7,65 ppm | singulet | 1H | $H_7$ | |
| δ : 11,05 ppm | signal | 1H | $NH^+$ | échangeable avec $D_2O$ |

**EXEMPLE 5 : 3-METHYL-6-[(4-(4-CHLOROPHENYL)PIPERAZIN-1-YL)METHYL] BENZOTHIAZOLINONE**

Il est identique à celui décrit pour l'obtention de l'exemple 2 en remplaçant la N-orthométhoxyphényl pipérazine par la N-parachlorophényl pipérazine et la 3-méthyl-6-chlorométhylbenzoxazolinone par la 3-méthyl-6-chlorométhylbenzothiazolinone. Le produit sous forme chlorhydrate est obtenu sous forme base dans l'acétate d'éthyle en présence de triéthylamine.

Temps de réaction : 2 jours
Masse molaire : 373,90 g.mol$^{-1}$ pour $C_{19}H_{20}ClN_3OS$
Point de fusion : 162-163°C
Rendement : 45 %
Solvant de recristallisation : méthanol
Rf : 0,7 Eluant : méthanol (saturé d'ammoniac)/chloroforme (1/9)

Spectrométrie dans l'infrarouge

| | |
|---|---|
| 2960-2740 $cm^{-1}$ | νCH |
| 1660 $cm^{-1}$ | νCO-S |
| 1590 $cm^{-1}$ | νC=C |

Spectrométrie de résonance magnétique nucléaire (300 MHz, DMSO/d6)

| | | | |
|---|---|---|---|
| δ : 2,50 ppm | massif | 4H | pipérazine |
| δ : 3,10 ppm | massif | 4H | pipérazine |
| δ : 3,40 ppm | singulet | 3H | $N-CH_3$ |
| δ : 3,55 ppm | singulet | 2H | $CH_2$ |
| δ : 6,90 ppm | massif | 2H | $H_4$, $H_5$ |
| δ : 7,30 ppm | massif | 4H | phényl |
| δ : 7,60 ppm | singulet | 1H | $H_7$ |

**<u>EXEMPLE 6</u> : 3-METHYL-6-[(4-(4-CHLOROPHENYL)PIPERAZIN-1-YL)METHYL] BENZOXAZOLINONE**

Il est identique à celui décrit pour l'obtention de l'exemple 2 en remplaçant la N-orthométhoxyphénylpipérazine par la N-parachlorophénylpipérazine. Le produit sous forme de chlorhydrate est transformé sous forme base dans l'acétate d'éthyle en présence de triéthylamine.

Temps de réaction : 3 jours
Masse molaire : 357,84 $g.mol^{-1}$ pour $C_{19}H_{20}ClN_3O_2$
Point de fusion : 165-166°C
Rendement : 65 %
Solvant de recristallisation : méthanol
Rf : 0,8 Eluant : méthanol (saturé d'ammoniac)/chloroforme (1/9)

Spectrométrie dans l'infrarouge

| | |
|---|---|
| 2960-2740 $cm^{-1}$ | νCH |
| 1760 $cm^{-1}$ | νCO-O |
| 1615 $cm^{-1}$ | νC=C |
| 1590 $cm^{-1}$ | νC=C |

Spectrométrie de résonance magnétique nucléaire (300 MHz, DMSO/d6)

| | | | |
|---|---|---|---|
| δ:2,50 ppm | massif | 4H | pipérazine |
| δ:3,10 ppm | massif | 4H | pipérazine |
| δ:3,35 ppm | singulet | 3H | $N-CH_3$ |
| δ: 3,55 ppm | singulet | 2H | $CH_2$ |
| δ:6,90 ppm | massif | 2H | $H_4$, $H_5$ |
| δ:7,20 ppm | massif | 4H | phényl |
| δ:7,30 ppm | singulet | 1H | $H_7$ |

**EXEMPLE 10 : 6-[(4-(2-FLUOROPHENYL)PIPERAZIN-1-YL)METHYL] BENZOXAZOLINONE**

Dans 50 $cm^3$ d'acétone, ajouter 0,010 mole de 6-chlorométhylbenzoxazolinone, 0,012 mole de N-orthofluorophényl pipérazine, 0,020 mole de triéthylamine et 0,001 mole d'iodure de potassium. Chauffer à reflux pendant 2 jours, sous agitation magnétique. Filtrer le résidu minéral et évaporer l'acétone. Ajouter 30 $cm^3$ d'acide chlorhydrique 1N et 20 $cm^3$ d'acétate d'éthyle. Laisser 30 minutes sous agitation magnétique. Filtrer le précipité obtenu, le laver à plusieurs reprises avec de l'acétate d'éthyle. Le produit sous forme chlorhydrate est obtenu sous forme base dans l'acétate d'éthyle en présence de triéthylamine. Sécher puis recristalliser le produit dans le toluène.

Masse molaire : 327,36 $g.mol^{-1}$ pour $C_{18}H_{18}FN_3O_2$
Point de fusion : 177-179°C
Rendement : 46 %
Rf : 0,4 Eluant : méthanol (saturé d'ammoniac)/chloroforme (1/9)

Spectrométrie dans l'infrarouge

| | |
|---|---|
| 3240 $cm^{-1}$ | νNH |
| 2960-2740 $cm^{-1}$ | νCH |
| 1760 $cm^{-1}$ | νCO-O |
| 1610 $cm^{-1}$ | νC=C |

Spectrométrie de résonance magnétique nucléaire (300 MHz, DMSO/d6)

| | | | |
|---|---|---|---|
| δ: 2,50 ppm | massif | 4H | pipérazine |
| δ: 3,00 ppm | massif | 4H | pipérazine |
| δ: 3,50 ppm | singulet | 2H | $CH_2$ |
| δ: 7,00 ppm | massif | 6H | $H_4$, $H_5$ et phényl |
| δ: 7,20 ppm | singulet | 1H | $H_7$ |
| δ: 11,50 ppm | singulet | 1H | NH échangeable avec $D_2O$ |

**EXEMPLE 11 : 3-[2-(4-(4-FLUOROBENZOYL)PIPERIDIN-1-YL)ETHYL]-6-[(4-(2-FLUOROPHENYL) PIPERAZIN-1-YL) METHYL] BENZO- -XAZOLINONE**

Dans 50 $cm^3$ de diméthyformamide anhydre, introduire 0,010 mole de 6-[(4-(orthofluorophényl)pipérazin-1-yl) méthyl]benzoxazolinone et 0,060 mole de carbonate de potassium. Agiter pendant 30 minutes à reflux et ajouter 0,012 mole du chlorhydrate de la 1-(2-chloroéthyl)-4-(parafluorobenzoyl)pipéridine. Chauffer à reflux pendant 3 heures, sous agitation magnétique. Refroidir, filtrer le solide minéral et verser le filtrat dans 100 $cm^3$ d'eau glacée. Filtrer le précipité formé, le laver à l'eau, le sécher puis le recristalliser dans l'éthanol absolu.

Masse molaire : 560,64 $g.mol^{-1}$ pour $C_{32}H_{34}F_2N_4O_3$
Point de fusion : 124-126°C
Rendement : 82 %
Rf : 0,7 Eluant : méthanol (saturé d'ammoniac)/chloroforme (1/9)

Spectrométrie dans l'infrarouge

| | |
|---|---|
| 2960-2740 $cm^{-1}$ | νCH |
| 1775 $cm^{-1}$ | νCO-O |
| 1670 $cm^{-1}$ | νCO |

(suite)

| | |
|---|---|
| 1600 $cm^{-1}$ | νC=C |

Spectrométrie de résonance magnétique nucléaire (300 MHz, DMSO/d6)

| | | | | |
|---|---|---|---|---|
| δ : 1,45 ppm | multiplet | 2H | pipéridine | |
| δ : 1,70 ppm | multiplet | 2H | pipéridine | |
| δ : 2,15 ppm | multiplet | 2H | pipéridine | |
| δ : 2,50 ppm | massif | 4H | pipérazine | |
| δ : 2,65 ppm | triplet | 2H | $CH_2$-pipéridine | $J_{e-d}$ = 5,95 Hz |
| δ : 3,00 ppm | massif | 6H | pipérazine et pipéridine | |
| δ : 3,35 ppm | multiplet | 1H | pipéridine-CO | |
| δ : 3,55 ppm | singulet | 2H | $CH_2$-pipérazine | |
| δ : 3,90 ppm | triplet | 2H | $CH_2$-$CH_2$-pipéridine | $J_{d-e}$ = 5,95 Hz |
| δ : 7,05 ppm | massif | 5H | $H_4$ et phényl(pipérazine) | |
| δ : 7,30 ppm | massif | 4H | $H_5$, $H_7$ et benzoyl(pipéridine) | |
| δ : 8,00 ppm | massif | 2H | benzoyl(pipéridine) | |

**EXEMPLE 12 : CHLORHYDRATE DE LA 6-[(4-(2-FLUOROPHENYL)PIPERAZIN-1-YL)METHYL] BENZOTHIAZOLINONE**

Dans 50 $cm^3$ d'acétone, ajouter 0,010 mole de 6-chlorométhylbenzothiazolinone, 0,012 mole de N-orthofluorophényl pipérazine, 0,020 mole de triéthylamine et 0,001 mole d'iodure de potassium. Chauffer à reflux pendant 2 jours, sous agitation magnétique. Filtrer le résidu minéral et évaporer l'acétone. Ajouter 30 $cm^3$ d'acide chlorhydrique 1N et 20 $cm^3$ d'acétate d'éthyle. Laisser 30 minutes sous agitation magnétique. Filtrer le précipité obtenu, le laver à plusieurs reprises avec de l'acétate d'éthyle. Sécher puis recristalliser le produit dans l'éthanol à 95°.

Masse molaire : 379,88 $g.mol^{-1}$ pour $C_{18}H_{19}ClFN_3OS$
Point de fusion : > 260°C
Rendement : 61 %
Rf : 0,5 Eluant : méthanol (saturé d'ammoniac)/chloroforme (1/9)

Spectrométrie dans l'infrarouge

| | |
|---|---|
| 3120 $cm^{-1}$ | νNH |
| 2940-2820 $cm^{-1}$ | νCH |
| 2740-2540 $cm^{-1}$ | $νNH^+$ |
| 1670 $cm^{-1}$ | νCO-S |
| 1610 $cm^{-1}$ | νC=C |

Spectrométrie de résonance magnétique nucléaire (300 MHz, DMSO/d6)

| | | | | |
|---|---|---|---|---|
| δ : 3,10 ppm | massif | 4H | pipérazine | |
| δ : 3,50 ppm | massif | 4H | pipérazine | |
| δ : 4,50 ppm | singulet | 2H | $CH_2$ | |
| δ : 7,10 ppm | massif | 5H | $H_4$ et phényl | |
| δ : 7,50 ppm | doublet | 1H | $H_5$ | $J_{5-4}$ = 8,20 Hz |

(suite)

| | | | | |
|---|---|---|---|---|
| δ : 7,80 ppm | singulet | 1H | $H_7$ | |
| δ : 9,70 ppm | signal | 1H | $NH^+$ | échangeable $D_2O$ |
| δ : 12,15 ppm | singulet | 1H | NH | échangeable $D_2O$ |

**EXEMPLE 13 : DICHLORHYDRATE DE LA 3-[2-(4-(4-FLUOROBENZOYL) PIPERIDIN-1-YL)ETHYL]-6-[(4-(2-FLUOROPHENYL) PIPERAZIN-1-YL) METHYL] BENZOTHIAZOLINONE**

Dans 50 $cm^3$ de diméthylformamide anhydre, introduire 0,010 mole de 6-[(4-(orthofluorophényl)pipérazin-1-yl)méthyl]benzothiazolinone et 0,060 mole de carbonate de potassium. Agiter pendant 30 minutes à reflux et ajouter 0,012 mole du chlorhydrate de la 1-(2-chloroéthyl)-4-(parafluorobenzoyl)pipéridine. Chauffer à reflux pendant 1 heure, sous agitation magnétique. Refroidir, filtrer le solide minéral et verser le filtrat dans 100 $cm^3$ d'eau glacée. Filtrer le précipité formé, le laver à l'eau puis le sécher. Solubiliser le produit dans le minimum d'acétone et ajouter 100 $cm^3$ d'éther anhydre saturé d'acide chlorhydrique gazeux. Filtrer le précipité, le laver à l'éther, le sécher puis le recristalliser dans l'éthanol absolu.

Masse molaire : 649,63 $g.mol^{-1}$ pour $C_{32}H_{36}Cl_2F_2N_4O_2S$
Point de fusion : 251-253°C
Rendement : 56 %
Rf : 0,6 Eluant : méthanol (saturé d'ammoniac)/chloroforme (1/9)

Spectrométrie dans l'infrarouge

| | |
|---|---|
| 3060-2800 $cm^{-1}$ | νCH |
| 2740-2280 $cm^{-1}$ | $νNH^+$ |
| 1670 $cm^{-1}$ | νCO-S et νCO |
| 1590 $cm^{-1}$ | νC=C |

Spectrométrie de résonance magnétique nucléaire (300 MHz, DMSO/d6)

| | | | | |
|---|---|---|---|---|
| δ : 2,00 ppm | massif | 4H | pipéridine | |
| δ : 3,20 ppm | massif | 4H | pipérazine | |
| δ : 3,45 ppm | massif | 11H | pipérazine, pipéridine, <u>$CH_2$</u>-pipéridine | |
| δ : 4,45 ppm | massif | 4H | <u>$CH_2$</u>-pipérazine, <u>$CH_2$</u>-<u>$CH_2$</u>pipéridine | |
| δ : 7,10 ppm | massif | 4H | <u>phényl</u>(pipérazine) | |
| δ : 7,40 ppm | massif | 2H | $H_4$, $H_5$ | |
| δ : 4,45 ppm | massif | 4H | <u>$CH_2$</u>-pipérazine | |
| δ : 7,80 ppm | massif | 2H | <u>benzoyl</u>(pipéridine) | |
| δ : 8,00 ppm | singulet | 1H | $H_7$ | |
| δ : 8,10 ppm | massif | 2H | benzoyl(pipéridine) | |
| δ: 11,25 ppm | signal | 1H | $NH^+$ | échangeable avec $D_2O$ |
| δ: 11,70 ppm | signal | 1H | $NH^+$ | échangeable avec $D_2O$ |

**EXEMPLE 14 : 3-METHYL-6-[(4-(BENZO[d]1,2-THIAZOLYL)PIPERAZIN-1-YL) METHYL]BENZOTHIAZOLINONE**

Dans 50 $cm^3$ d'acétone, ajouter 0,010 mole de 3-méthyl-6-chlorométhyl benzothiazolinone, 0,012 mole de 1-(3-benzisothiazolyl)pipérazine, 0,020 mole de triéthylamine et 0,001 mole d'iodure de potassium. Chauffer à reflux, sous agitation magnétique, pendant 2 jours. Filtrer le résidu minéral et évaporer l'acétone. Ajouter 30 $cm^3$ d'acide chlorhydrique 1N et 20 $cm^3$ d'acétate d'éthyle. Laisser 30 minutes sous agitation magnétique. Filtrer le précipité obtenu, le laver à plusieurs reprises avec de l'acétate d'éthyle. Le produit sous forme chlorhydrate est passé sous forme base

dans l'acétate d'éthyle en présence de triéthylamine. Sécher le produit puis le recristalliser dans le méthanol.

Masse molaire : 396,53 g.mol$^{-1}$ pour $C_{20}H_{20}N_4OS_2$
Point de fusion : 163-165°C
Rendement : 31 %
Rf : 0,8 Eluant : méthanol (saturé d'ammoniac)/chloroforme (1/9)

Spectrométrie dans l'infrarouge

| | |
|---|---|
| 2980-2760 cm$^{-1}$ | νCH |
| 1665 cm$^{-1}$ | νCO-S |
| 1580 cm$^{-1}$ | ν C=C |

Spectrométrie de résonance magnétique nucléaire (300 MHz, DMSO/d6)

| | | | |
|---|---|---|---|
| δ:2,60 ppm | massif | 4H | pipérazine |
| δ: 3,40 ppm | massif | 7H | N-$CH_3$, et pipérazine |
| δ: 3,60 ppm | singulet | 2H | $CH_2$ |
| δ: 7,30 ppm | doublet | 1H | $H_4$ $J_{4-5}$ = 8,00 Hz |
| δ: 7,40 ppm | doublet | 1H | $H_5$ $J_{5-4}$=8,00 Hz |
| δ: 7,50 ppm | massif | 2H | benzisothiazole |
| δ: 7,65 ppm | singulet | 1H | $H_7$ |
| δ: 8,05 ppm | massif | 2H | benzisothiazole |

**<u>EXEMPLE 15</u> : 3-METHYL-6-[(4-(BENZO[d]1,2-THIAZOLYL)PIPERAZIN-1-YL) METHYL]BENZOXAZOLINONE**

Dans 50 cm$^3$ d'acétone, ajouter 0,010 mole de 3-méthyl-6-chlorométhyl benzoxazolinone, 0,012 mole de 1-(3-benzisothiazolyl)pipérazine, 0,020 mole de triéthylamine et 0,001 mole d'iodure de potassium. Chauffer à reflux, sous agitation magnétique, pendant 3 jours. Filtrer le résidu minéral et évaporer l'acétone. Ajouter 30 cm$^3$ d'acide chlorhydrique 1N et 20 cm$^3$ d'acétate d'éthyle. Laisser 30 minutes sous agitation magnétique. Filtrer le précipité obtenu, le laver à plusieurs reprises avec de l'acétate d'éthyle. Le produit sous forme chlorhydrate est transformé sous forme base dans l'acétate d'éthyle en présence de triéthylamine. Sécher le produit puis le recristalliser dans l'éthanol absolu.

Masse molaire : 380,47 g.mol$^{-1}$ pour $C_{20}H_{20}N_4O_2S$
Point de fusion : 193-194°C
Rendement 72 %
Rf : 0,6 Eluant : méthanol (saturé d'ammoniac)/chloroforme (1/9)

Spectrométrie dans l'infrarouge

| | |
|---|---|
| 3080-2760 cm$^{-1}$ | νCH |
| 1760 cm$^{-1}$ | νCO-O |
| 1620 cm$^{-1}$ | ν C=C |
| 1590 cm$^{-1}$ | ν C=C |

Spectrométrie de résonance magnétique nucléaire (300 MHz, DMSO/d6)

| | | | |
|---|---|---|---|
| δ : 2,60 ppm | massif | 4H | pipérazine |
| δ : 3,35 ppm | singulet | 3H | N-$CH_3$ |
| δ : 3,50 ppm | massif | 4H | pipérazine |
| δ : 3,60 ppm | singulet | 2H | $CH_2$ |
| δ : 7,20 ppm | massif | 2H | $H_4$, $H_5$ |
| δ : 7,30 ppm | singulet | 1H | $H_7$ |
| δ : 7,50 ppm | massif | 2H | benzisothiazole |
| δ : 8,05 ppm | massif | 2H | benzisothiazole |

**EXEMPLE 17 : DICHLORHYDRATE DE LA 3-{2-[4-(4',4"-DIFLUORO BENZHYDRYLIDENE)PIPERIDIN-1-YL] ETHYL}-6-[(4-(2-FLUOROPHENYL)PIPERAZIN-1-YL)METHYL]BENZOXAZOLINONE**

En procédant comme dans l'exemple 11 mais en remplaçant la 1-(2-chloroéthyl)-4-(parafluorobenzoyl)pipéridine par la 1-(2-chloroéthyl)-4-(4',4"-difluorobenzhydrylidene) pipéridine, on obtient le produit du titre.
En procédant de la même façon que dans les exemples 1 à 17 mais en utilisant les matières premières appropriées, on obtient les composés des exemples 18 à 26 :

**EXEMPLE 18 : CHLORHYDRATE DE LA 3-METHYL-6-{[4-(4-AMINOSULFONYLPHENYL)PIPERAZIN-1-YL] METHYL} BENZOXAZOLINONE**

**EXEMPLE 19 : CHLORHYDRATE DE LA 3-METHYL-6-{[4-(4-AMINOSULFONYLPHENYL)PIPERAZIN-1-YL] METHYL} BENZOTHIAZOLINONE**

**EXEMPLE 21 : CHLORHYDRATE DE LA 3-METHYL-6-{[4-(3-METHOXYPHENYL)PIPERAZIN-1-YL]METHYL} BENZOTHIAZOLINONE**

Point de fusion : 208-210°C

**EXEMPLE 22 : CHLORHYDRATE DE LA 3-METHYL-6-{[4-(3-METHOXYPHENYL)PIPERAZIN-1-YL]METHYL) BENZOXAZOLINONE**

Point de fusion : 227-229°C

**EXEMPLE 25 : CHLORHYDRATE DE LA 3-METHYL-6-{[4-(4-METHOXYPHENYL)PIPERAZIN-1-YL]METHYL} BENZOTHIAZOLINONE**

Point de fusion : >260°C

**EXEMPLE 26 : CHLORHYDRATE DE LA 3-METHYL-6-{[4-(4-METHOXYPHENYL)PIPERAZIN-1-YL]METHYL} BENZOXAZOLINONE**

Point de fusion : >250°C

**ETUDE PHARMACOLOGIQUE DES DERIVES DE L'INVENTION**

**EXEMPLE A: TEST D'AFFINITE IN VITRO POUR LES RECEPTEURS $D_4$, $D_2$, et 5-$HT_{1A}$**

Les tests d'affinité in vitro pour les récepteurs 5-$HT_{1A}$, $D_4$ et $D_2$ ont été réalisés selon des techniques classiques de binding.
Les résultats de ces études montrent que les composés de l'invention possèdent un $K_i$ de l'ordre de $10^{-7}$ M vis à vis des récepteurs 5-$HT_{1A}$ et $D_2$, soit environ 100 fois moins affin que les composés de la demande EP 478446.
L'affinité pour les récepteurs $D_{4.4}$ a été déterminée par des expériences de compétition avec le [$^3$H]spiperone (NEN, les Ulis, France). Les membranes préparées à partir de cellules CHO transfectées avec le récepteur humain

$D_{4.4}$ ont été achetées auprès de Receptor Biology Inc. (MD, USA). Les membranes (30 µg de protéine membranaire) sont incubées en triple avec 0,5 nM de [$^3$H]spiperone et le ligand froid dans un volume final de 0,5 ml, pendant 60 min à 25°C. Le tampon d'incubation contient 50 nM de TRIS-HCl (pH 7,4), 120 mM de NaCl, 5 mM de KCl, 5 mM de $MgCl_2$ et 1mM de EDTA. La fixation non-spécifique est déterminée avec 10 µM d'haloperidol. A la fin de l'incubation, le milieu d'incubation est filtré au travers de filtres WHATMAN GF/B imprégnés avec 0,1 % de polyéthylénimine et lavés trois fois avec 2 ml de tampon refroidi. La radioactivité retenue sur les filtres est déterminée par comptage du liquide de scintillation. Les isothermes de binding sont analysées par régression non-linéaire utilisant le logiciel 'PRISM' (GraphPad Software Inc., S. Diego, USA) pour déterminer des valeurs de $IC_{50}$. Elles sont converties en constante de dissociation ($K_1$) par l'intermédiaire de l'équation de Cheng-Prusoff:

$$K_i = IC_{50}/\{[L/K_d)-1\}$$

dans laquelle L est la concentration de [$^3$H]spiperone libre et $K_d$ est la constante de dissociation de [$^3$H]spiperone du récepteur humain $D_{4.4}$ (70 pM).

Pour les récepteurs $D_{4.4}$ l'affinité $K_i$ est de l'ordre de $10^{-8}$ - $10^{-9}$ alors que les composés de la demande EP 478446 n'avaient qu'une affinité de l'ordre de $10^{-6}$ M.

**EXEMPLE B : DETERMINATION DE L'EFFICACITE AU NIVEAU DES RECEPTEURS HUMAINS $D_{4.4}$**

L'efficacité a été déterminée en mesurant l'activation des protéines G par la stimulation de la fixation du [$^{35}$S] GTPγS (NEN, Les Ulis, France). Les membranes préparées à partir de cellules CHO transfectées avec le récepteur humain $D_{4.4}$ ont été achetées auprès de Receptor Biology Inc. (MD, USA). Les membranes (50 µg de protéine membranaire) sont incubées en triple avec 0,1 nM de [$^{35}$S] GTPγS et le ligand froid dans un volume final de 0,5 ml, pendant 20 min à 25°C. Le tampon d'incubation contient 20 mM de HEPES (pH 7,4), 3 µM de GDP, 3 mM de $MgCl_2$ et 100 mM de NaCl. A la fin de l'incubation le milieu d'incubation est filtré au travers de filtres WHATMAN GF/B imprégnés d'eau et lavés trois fois avec 2 ml de tampon refroidi. La radioactivité retenue sur les filtres est déterminée par comptage du liquide de scintillation. Les isothermes de binding sont analysées par régression non-linéaire utilisant le logiciel 'PRISM' (GraphPad Software Inc., S. Diego, USA) pour déterminer des valeurs de $EC_{50}$ et d'efficacité ($E_{max}$). L'efficacité est exprimée comme un pourcentage de la stimulation de la fixation du [$^{35}$S]GTPγS induite par la dopamine.

Pour les tests d'antagonisme, les membranes sont pré-incubées avec l'antagoniste et une concentration fixe de dopamine pendant 30 min avant d'ajouter le [$^{35}$S]GTPγS. Les valeurs de $IC_{50}$ sont converties en constante de puissance antagoniste ($K_b$) par l'intermédiaire de l'équation suivante :

$$K_b = IC_{50}/\{[Antagoniste]/EC_{50}]+1\}$$

dans laquelle [Antagoniste] est la concentration d'antagoniste
et $EC_{50}$ est le $EC_{50}$ déterminé dans l'absence d'antagoniste (dopamine seule).

Pour l'exemple 1, le $K_b$ est de 2.39 ± 1.10 nM.
Pour l'exemple 3, le $K_b$ est de 16.1 ± 5.0 nM.

**EXEMPLE C : TOXICITE AIGUE**

La toxicité aigüe a été appréciée après administration orale à des lots de 8 souris (26 ± 2 grammes) d'une dose de 650 mg.kg$^{-1}$. Les animaux ont été observés à intervalles réguliers au cours de la première journée et quotidiennement pendant les deux semaines suivant le traitement.

Il apparaît que la plupart des composés de l'invention sont totalement atoxiques. La plupart d'entre eux n'entraîne aucun décés après administration à une dose de 650 mg.kg$^{-1}$ et on ne constate généralement pas de troubles après administration de cette dose.

**COMPOSITIONS PHARMACEUTIQUES**

Comprimés destinés au traitement des affections psychiques dosés à 1 mg de 3-méthyl 6-[(4-(2-fluorophényl) pipérazin-1-yl)méthyl]benzothiazolinone, chlorhydrate.

Formule de préparation pour 1000 comprimés :

| | |
|---|---|
| 3-méthyl 6-[(4-(2-fluorophényl)pipérazin-1-yl) méthyl]benzoxathiazolinone, chlorhydrate | 1 g |
| Amidon de blé | 20 g |
| Amidon de maïs | 20 g |
| Lactose | 65 g |
| Stéarate de magnésium | 2 g |
| Silice | 1 g |
| Hydroxypropylcellulose | 2 g |

**Revendications**

**1.** Dérivés de formule générale (I):

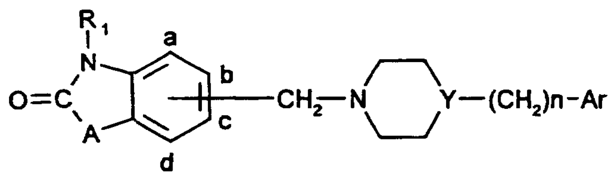

(I)

dans laquelle :

$R_1$ représente un atome d'hydrogène ou un groupement alkyle inférieur, ou encore
$R_1$ représente un groupement

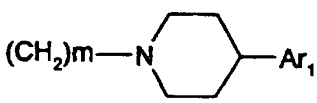

dans laquelle m représente un entier compris inclusivement entre 1 et 4 et Ar1 représente

. ou bien un groupement CO-Ar2 où Ar2 représente un noyau phényle non substitué ou substitué par un ou plusieurs radicaux choisis parmi halogène, hydroxy, alkyle inférieur, trifluorométhyle ou alkoxy inférieur,
. ou bien un groupement $=C-(Ar_2)_2$ où $Ar_2$ a la même signification que précédemment,

n représente 0 ou 1
A représente un atome d'oxygène ou de soufre
Y représente un groupement CH ou un atome d'azote
Ar représente un groupement phényle ou naphtyle éventuellement substitué par un, deux ou trois groupements choisis parmi halogène, hydroxy, alkoxy inférieur, alkyle inférieur, alkoxy inférieur-alkyle inférieur ou trifluorométhyle, ou aminosulfonyle ou Ar représente un groupement pyridyle, pyrimidinyle, ou 3-(benzo[d]-1,2-thiazolyle) encore appelé 3-benzisothiazolyle :

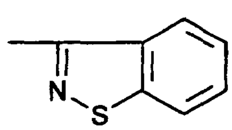

le cas échéant leurs isomères purs ou en mélange ainsi que leurs sels d'addition à un acide pharmaceutiquement acceptable, ou une base pharmaceutiquement acceptable lorsque $R_1$ = H,
étant entendu que, sauf indication contraire,

- les termes "alkyle inférieur" et "alkoxy inférieur" correspondent à des groupements linéaires ou ramifiés contenant de 1 à 6 atomes de carbone.

**2.** Composés de formule (I) selon la revendication 1 pour lesquels pris ensemble ou séparément :

. $R_1$ est un atome d'hydrogène ou un groupement méthyle ou un groupement

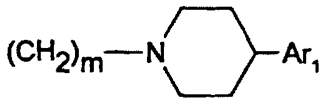

pour lequel m vaut 2 et $Ar_1$ représente ou bien un groupement

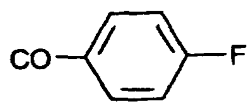

ou encore un groupement

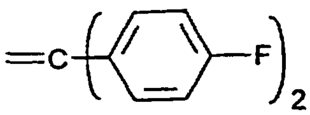

. A est un atome de soufre

. A est un atome d'oxygène

. la chaîne latérale est greffée en position c
. n représente 0
. Y représente un atome d'azote
. Ar représente un groupement phényle substitué par un atome de fluor ou encore par un atome de chlore ou encore par un groupement méthoxy, ainsi que leurs isomères purs ou en mélange ainsi que leurs sels d'addition à un acide pharmaceutiquement acceptable ou une base pharmaceutiquement acceptable lorsque $R_1$ = H.

**3.** Dérivé de formule (I) selon la revendication 1 choisi parmi :

. la 3-méthyl-6-{[4-(2-méthoxyphényl)pipérazin-1-yl]méthyl}benzoxazolinone, ainsi que ses sels d'addition à un acide pharmaceutiquement acceptable,

. la 3-méthyl-6-{[4-(2-méthoxyphényl)pipérazin-1-yl]méthyl}benzothiazolinone, ainsi que ses sels d'addition à un acide pharmaceutiquement acceptable,

. la 3-méthyl-6-{[4-(2-fluorophényl)pipérazin-1-yl]méthyl}benzothiazolinone, ainsi que ses sels d'addition à un acide pharmaceutiquement acceptable,

. la 3-méthyl-6-{[4-(2-fluorophényl) pipérazin-1-yl]méthyl}benzoxazolinone, ainsi que ses sels d'addition à un acide pharmaceutiquement acceptable,

- la 3-méthyl-6-{[4-(4-chlorophényl)pipérazin-1-yl]méthyl}benzoxazolinone ainsi que ses sels d'addition à un acide pharmaceutiquement acceptable,

- la 3-méthyl-6-{[4-(4-chlorophényl)pipérazin-1-yl]méthyl}benzothiazolinone ainsi que ses sels d'addition à un acide pharmaceutiquement acceptable,

- la 6-{[4-(2-fluorophényl)pipérazin-1-yl]méthyl}benzoxazolinone ainsi que ses sels d'addition à un acide ou une base pharmaceutiquement acceptables,

- la 6-{[4-(2-fluorophényl)pipérazin-1-yl]méthyl}benzothiazolinone ainsi que ses sels d'addition à un acide ou une base pharmaceutiquement acceptables,

- la 3-{2-[4-(4-fluorobenzoyl)pipéridyl]éthyl}-6-{[4-(2-fluorophényl)pipérazin-1-yl] méthyl} benzoxazolinone ainsi que ses sels d'addition à un acide pharmaceutiquement acceptable,

- la 3-{2-[4-(4',4"-difluorobenzhydrylidene)pipéridin-1-yl]éthyl}-6-{[4-(2-fluorophényl) pipérazin-1-yl]méthyl} benzoxazolinone ainsi que ses sels d'addition à un acide pharmaceutiquement acceptable,

- la 3-{2-[4-(4-fluorobenzoyl)pipéridin-1-yl]éthyl}-6-{[4-(2-fluorophényl)pipérazin-1-yl] méthyl}benzothiazolinone ainsi que ses sels d'addition à un acide pharmaceutiquement acceptable,

- la 3-méthyl-6-{[4-(benzold]1,2-thiazolyl)pipérazin-1-yl]méthyl}benzoxazolinone, ainsi que ses sels d'addition à un acide pharmaceutiquement acceptable,

- la 3-méthyl-6-{[4-(benzo[d]1,2-thiazolyl)pipérazin-1-yl]méthyl}benzothiazolinone, ainsi que ses sels d'addition à un acide pharmaceutiquement acceptable,

- la 3-méthyl-6-{[4-(4-aminosulfonylphényl)pipérazin-1-yl]méthyl}benzoxazolinone ainsi que ses sels d'addition à un acide pharmaceutiquement acceptable,

- la 3-méthyl-6-{[4-(4-aminosulfonylphényl)pipérazin-1-yl]méthyl}benzothiazolinone ainsi que ses sels d'addition à un acide pharmaceutiquement acceptable,

- la 3-méthyl-6-{[4-(3-méthoxyphényl}pipérazin-1-yl]méthyl}benzoxazolinone ainsi que ses sels d'addition à un acide pharmaceutiquement acceptable,

- la 3-méthyl-6-{[4-(4-méthoxyphényl)pipérazin-1-yl]méthyl}benzothiazolinone ainsi que ses sels d'addition à un acide pharmaceutiquement acceptable,

- la 3-méthyl-6-{[4-méthoxyphényl)pipérazin-1-yl]méthyl}benzoxazolinone ainsi que ses sels d'addition à un acide pharmaceutiquement acceptable,

4. Composé de formule (I) selon la revendication 1 qui est la 3-méthyl-6{[4-(2-fluorophényl)pipérazin-1-yl)]méthyl} benzothiazolinone ainsi que ses sels d'addition à un acide pharmaceutiquement acceptable.

5. Composé de formule (I) selon la revendication 1 qui est la 3-méthyl-6{[4-(4-chlorophényl)pipérazin-1-yl)]méthyl} benzoxazolinone ainsi que ses sels d'addition à un acide pharmaceutiquement acceptable.

6. Procédé de préparation des composés de formule (I) selon la revendication 1, caractérisé

   - d'une part, lorsque dans le composé de formule (I) que l'on veut obtenir le groupement $R_1$ représente un groupement alkyle inférieur, en ce que l'on fait réagir un composé de formule (II):

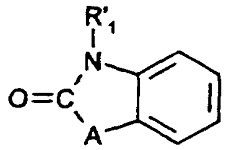

(II)

dans laquelle A a la même signification que précédemment et $R'_1$ représente un groupement alkyle inférieur avec l'hexaméthylène tétramine, préférentiellement en milieu acide pour conduire à un produit de formule (III) :

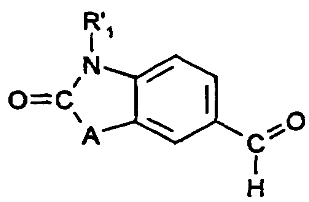

(III)

où $R'_1$ et A ont la même signification que précédemment
que l'on traite par un agent d'hydrogénation pour conduire à un produit de formule (IV):

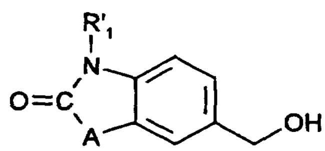

(IV)

où $R'_1$ et A ont la même signification que précédemment,

- d'autre part, lorsque dans le composé de formule (I) que l'on souhaite obtenir $R_1$ est différent du groupement alkyle inférieur en ce que l'on traite un dérivé de formule (V):

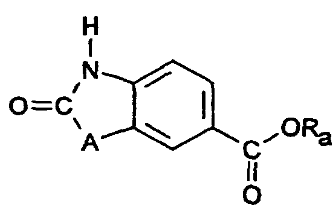

(V)

où $R_a$ représente un groupement alkyle inférieur et A a la même signifization que précédemment, par un agent d'hydrogénation pour obtenir un dérivé de formule (IVb):

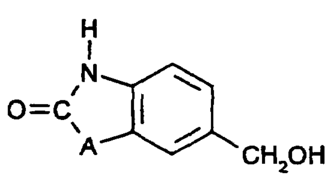

(IVb)

où A a la même signification que précédemment

- dérivé de formule (IV) ou (IVb) que l'on traite par un agent d'halogénation pour conduire à un produit de formule (VI) :

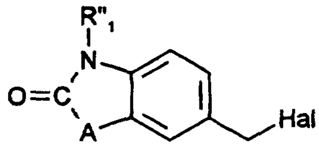

(VI)

où $R''_1$ représente un atome d'hydrogène ou un groupement alkyle inférieur et A a la même signification que précédemment et Hal représente un atome d'halogène
que l'on traite par une amine de formule (VII) :

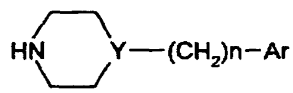

(VII)

où Y, n et Ar ont la même signification que dans la formule (I)
pour obtenir un produit de formule (I/a)

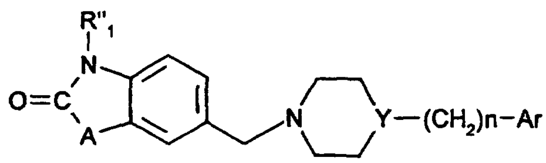

(I/a)

cas particulier des dérivés de formule (I) ; $R''_1$, A, Y, n et Ar ayant la même signification que précédemment dérivé de formule I/a, qui, lorsque dans le dérivé de formule (I) que l'on souhaite obtenir $R_1$ ne représente ni un atome d'hydrogène ni un groupement alkyle inférieur, est traité par un dérivé de formule (IX) :

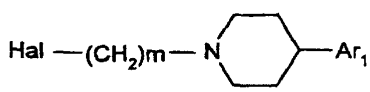

(IX)

où Hal représente un atome d'halogène, et m et $Ar_1$ ont la même définition que dans la formule (I)
pour conduire à un dérivé de formule I/c :

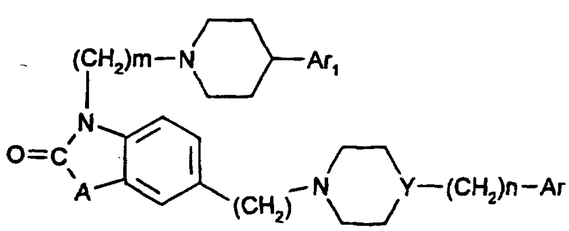

(I/c)

cas particulier des dérivés de formule (I) pour lesquels $R_1$ représente un groupement

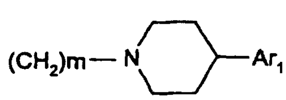

le dérivé de formule I/a ou I/c ainsi obtenu étant le cas échéant

- purifié par une ou plusieurs méthodes choisies parmi cristallisation, chromatographie, extraction, passage sur résine ou charbon
- séparé le cas échéant, sous forme pure ou sous forme de mélange, en ses isomères optiques,

- et si on le désire salifié par un acide ou une base pharmaceutiquement acceptable,

étant entendu que le terme "alkyle inférieur" correspond à des groupements linéaires ou ramifiés contenant de 1 à 6 atomes de carbone.

**7.** Composés de formule III, IV, IVb et VI selon la revendication 6 utiles en tant qu'intermédiaires de synthèse des dérivés de formule (I) à l'exception du dérivé de formule III pour lequel A représente un atome d'oxygène et $R'_1$ un groupement méthyle.

**8.** Composés selon la revendication 7 qui sont :

- la 3-méthyl-6-formyl benzothiazolinone,
- la 3-méthyl-6-hydroxyméthyl benzoxazolinone,
- la 3-méthyl-6-hydroxyméthyl benzothiazolinone,
- la 6-hydroxyméthyl benzoxazolinone,
- la 6-hydroxyméthyl benzothiazolinone,
- les 3-méthyl-6-halogénométhyl benzoxazolinones,
- les 3-méthyl-6-halogénométhyl benzothiazolinones,
- les 6-halogénométhyl benzoxazolinones,
- les 6-halogénométhyl benzothiazolinones.

**9.** Compositions pharmaceutiques contenant comme principe actif au moins un composé selon l'une des revendications 1 à 5 en combinaison avec un ou plusieurs excipients ou véhicules pharmaceutique acceptables.

**10.** Compositions pharmaceutiques selon la revendication 9 utilisable dans le traitement des affections du comportement psychique et plus particulièrement dans les psychoses, la schizophrénie, la modulation de l'humeur et des fonctions cognitives, dans l'anxiété, la dépression, l'abus de drogues, les états impulsifs, les troubles mnémocognitifs et les états migraineux.

## Claims

**1.** Compounds of general formula (I):

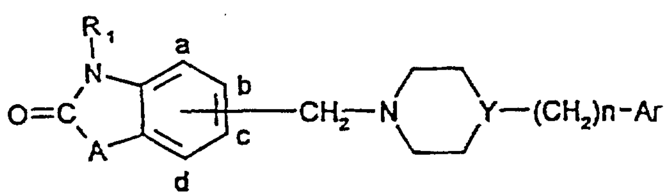

(I)

wherein:

$R_1$ represents a hydrogen atom or a lower alkyl group, or
$R_1$ represents a group

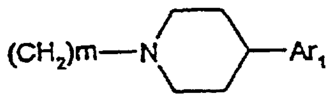

wherein m represents an integer from 1 to 4 inclusive and $Ar_1$ represents

- either a group $CO-Ar_2$ wherein $Ar_2$ represents a phenyl ring that is unsubstituted or substituted by one or more radicals selected from halogen, hydroxy, lower alkyl, trifluoromethyl and lower alkoxy,
- or a group $=C-(Ar_2)_2$ wherein $Ar_2$ is as defined hereinbefore,

n is 0 or 1,
A represents an oxygen or sulphur atom,
Y represents a CH group or a nitrogen atom
Ar represents a phenyl or naphthyl group optionally substituted by one, two or three groups selected from halogen, hydroxy, lower alkoxy, lower alkyl, lower alkoxylower alkyl and trifluoromethyl, and aminosulphonyl, or Ar represents a pyridyl group, a pyrimidinyl group or a 3-(benzo[d]-1,2-thiazolyl) group, also called 3-benzisothiazolyl:

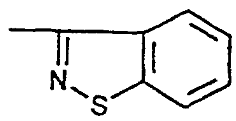

where appropriate to their isomers pure or in a mixture, and to addition salts thereof with a pharmaceutically acceptable acid, or with a pharmaceutically acceptable base when $R_1$=H,
it being understood, unless indicated otherwise, that

- the terms "lower alkyl" and "lower alkoxy" correspond to linear or branched groups containing from 1 to 6 carbon atoms,

**2.** Compounds of formula (I) according to claim 1 wherein, taken together or separately:

- $R_1$ is a hydrogen atom, a methyl group or a group

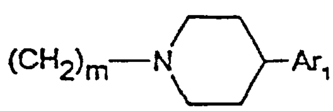

wherein m is 2 and $Ar_1$ represents either a group

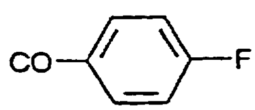

or a group

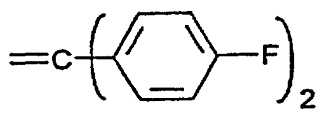

- A is a sulphur atom,

- A is an oxygen atom,

- the side chain is attached in the c position
- n is 0
- Y represents a nitrogen atom
- Ar represents a phenyl group substituted by a fluorine atom or by a chlorine atom or by a methoxy group, and to their isomers pure or in a mixture, and to addition salts thereof with a pharmaceutically acceptable acid or with a pharmaceutically acceptable base when $R_1$ = H.

**3.** Compound of formula (I) according to claim 1 selected from:

- 3-methyl-6-{[4-(2-methoxyphenyl)piperazin-1-yl]methyl}benzoxazolinone, and addition salts thereof with a pharmaceutically acceptable acid,

- 3-methyl-6-{[4-(2-methoxyphenyl)piperazin-1-yl]methyl}benzothiazolinone, and addition salts thereof with a pharmaceutically acceptable acid,

- 3-methyl-6-{[4-(2-fluorophenyl)piperazin-1-yl]methyl}benzothiazolinone, and addition salts thereof with a pharmaceutically acceptable acid,

- 3-methyl-6-{[4-(2-flurophenyl)piperazin-1-yl]methyl}benzoxazolinone, and addition salts thereof with a pharmaceutically acceptable acid,

- 3-methyl-6-{[4-(4-chlorophenyl)piperazin-1-yl]methyl}benzoxazolinone, and addition salts thereof with a pharmaceutically acceptable acid,

- 3-methyl-6-{[4-(4-chlorophenyl)piperazin-1-yl]methyl)benzothiazolinone, and addition salts thereof with a pharmaceutically acceptable acid,

- 6-{[4-(2-fluorophenyl)piperazin-1-yl]methyl}benzoxazolinone, and addition salts thereof with a pharmaceutically acceptable acid or base,

- 6-{[4-(2-fluorophenyl)piperazin-1-yl]methyl}benzothiazolinone, and addition salts thereof with a pharmaceutically acceptable acid or base,

- 3-{2-[4-(4-fluorobenzoyl)piperidyl]ethyl}-6-{[4-(2-fluorophenyl)piperazin-1-yl]methyl}-benzoxazolinone, and addition salts thereof with a pharmaceutically acceptable acid,

- 3-{2-[4-(4',4''-difluorobenzhydrylidene)piperidin-1-yl]ethyl}-6-{[4-(2-fluorophenyl)-piperazin-1-yl)methyl}benzoxazolinone, and addition salts thereof with a pharmaceutically acceptable acid,

- 3-{2-[4-(4-fluorobenzoyl)piperidin-1-yl]ethyl}-6-{[4-(2-fluorophenyl)piperazin-1-yl]methyl}benzothiazolinone, and addition salts thereof with a pharmaceutically acceptable acid,

- 3-methyl-6-{[4-(benzo[d]-1,2-thiazolyl)piperazin-1-yl]methyl)benzoxazolinone, and addition salts thereof with a pharmaceutically acceptable acid,

- 3-methyl-6-{[4-(benzo[d]-1,2-thiazolyl)piperazin-1-yl]methyl}benzothiazolinone, and addition salts thereof with a pharmaceutically acceptable acid,

. 3-methyl-6-{[4-(4-aminosulphonylphenyl)piperazin-1-yl]methyl}benzoxazolinone, and addition salts thereof with a pharmaceutically acceptable acid,

. 3-methyl-6-{[4-(4-aminosulphonylphenyl)piperazin-1-yl]methyl}benzothiazolinone, and addition salts thereof with a pharmaceutically acceptable acid,

. 3-methyl-6-{[4-(3-methoxyphenyl)piperazin-1-yl]methyl}benzoxazolinone, and addition salts thereof with a pharmaceutically acceptable acid,

. 3-methyl-6-{[4-(4-methoxyphenyl)piperazin-1-yl]methyl)benzothiazolinone, and addition salts thereof with a pharmaceutically acceptable acid,

. 3-methyl-6-{[4-(4-methoxyphenyl)piperazin-1-yl]methyl}benzoxazolinone, and addition salts thereof with a pharmaceutically acceptable acid.

**4.** Compound of formula (I) according to claim 1 which is 3-methyl-6-{[4-(2-fluorophenyl)piperazin-1-yl)]methyl}benzothiazolinone and addition salts thereof with a pharmaceutically acceptable acid.

**5.** Compound of formula (I) according to claim 1 which is 3-methyl-6-{[4-(4-chlorophenyl)piperazin-1-yl)]methyl}benzoxazolinone and addition salts thereof with a pharmaceutically acceptable acid.

**6.** Process for the preparation of compounds of formula (I) according to claim 1, characterised in that

. on the one hand when in the desired compound of formula (I) the group $R_1$ represents a lower alkyl group, a compound of formula (II):

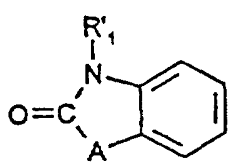

(II)

wherein A is as defined hereinbefore and $R'_1$ represents a lower alkyl group, is reacted with hexamethylenetetramine, preferably in an acidic medium, to yield a product of formula (III):

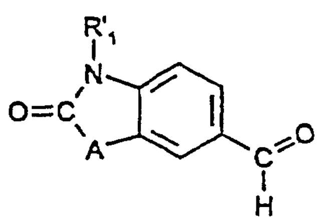

(III)

wherein $R'_1$ and A are as defined hereinbefore,
which is treated with a hydrogenating agent to yield a product of formula (IV):

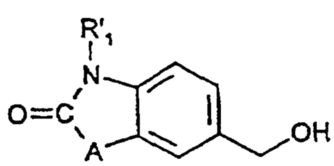

(IV)

wherein $R'_1$ and A are as defined hereinbefore,

. and when in the desired compound of formula (I) $R_1$ is other than a lower alkyl group, a compound of formula

(V):

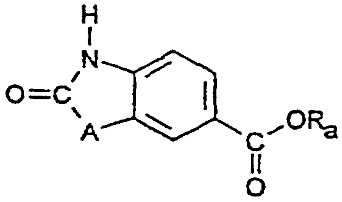

(V)

wherein $R_a$ represents a lower alkyl group and A is as defined hereinbefore, is treated with a hydrogenating agent to obtain a compound of formula (IVb):

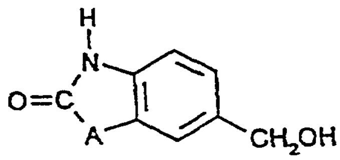

(IVb)

wherein A is as defined hereinbefore

. which compound of formula (IV) or (IVb) is treated with a halogenating agent to yield a product of formula (VI):

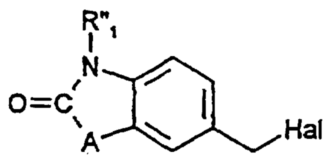

(VI)

wherein $R''_1$ represents a hydrogen atom or a lower alkyl group and A is as defined hereinbefore and Hal represents a halogen atom,
which is treated with an amine of formula (VII):

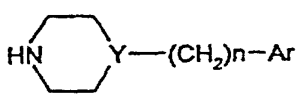

(VII)

wherein Y, n and Ar are as defined for formula (I)
to obtain a product of formula (I/a)

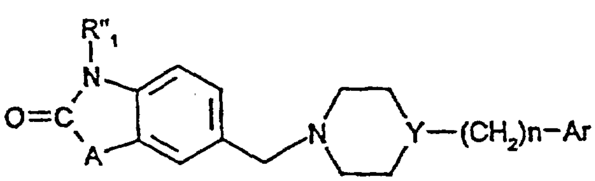

(I/a)

a particular case of the compounds of formula (I), with $R''_1$, A, Y, n and Ar being as defined hereinbefore, which compound of formula I/a, when in the desired compound of formula (I) $R_1$ represents neither a hydrogen atom nor a lower alkyl group, is treated with a compound of formula (IX):

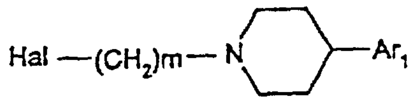

(IX)

wherein Hal represents a halogen atom, and m and $Ar_1$ are as defined for formula (I), to yield a compound of formula (I/c):

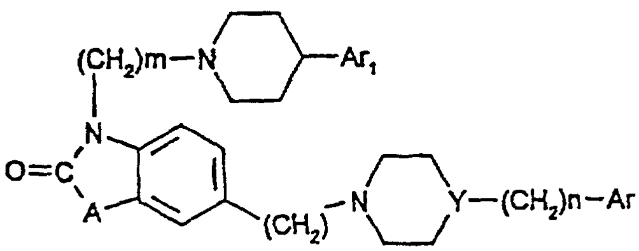

(I/c)

a particular case of the compounds of formula (I) wherein $R_1$ represents a group

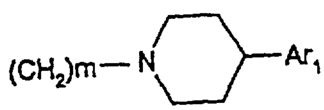

which compound of formula I/a or I/c so obtained is, where appropriate,

- purified by one or more methods selected from crystallisation, chromatography, extraction, passage over resin or carbon,
- separated, where appropriate, in pure form or in the form of a mixture, into its optical isomers, and
- if desired converted into a salt with a pharmaceutically acceptable acid or base,

it being understood that the term "lower alkyl" corresponds to linear or branched groups containing from 1 to 6 carbon atoms.

**7.** Compounds of formulae III, IV, IVb and VI according to claim 6 for use as intermediates in the synthesis of compounds of formula (I) with the exception of the compound of formula III wherein A represents an oxygen atom and $R'_1$ represents a methyl group.

**8.** Compounds according to claim 7 which are:

- 3-methyl-6-formylbenzothiazolinone,
- 3-methyl-6-hydroxymethylbenzoxazolinone,
- 3-methyl-6-hydroxymethylbenzothiazolinone,
- 6-hydroxymethylbenzoxazolinone,
- 6-hydroxymethylbenzothiazolinone,
- 3-methyl-6-halomethylbenzoxazolinones,
- 3-methyl-6-halomethylbenzothiazolinones,

- 6-halomethylbenzoxazolinones,
- 6-halomethylbenzothiazolinones.

**9.** Pharmaceutical compositions comprising as active ingredient at least one compound according to any one of claims 1 to 5 in combination with one or more pharmaceutically acceptable excipients or carriers.

**10.** Pharmaceutical compositions according to claim 9 for use in the treatment of psychological behaviour disorders and more especially in psychoses, schizophrenia, modulation of mood and of cognitive functions, anxiety, depression, drug abuse, impulsive states, mnemocognitive disorders and migraine states.

**Patentansprüche**

**1.** Derivate der allgemeinen Formel (I):

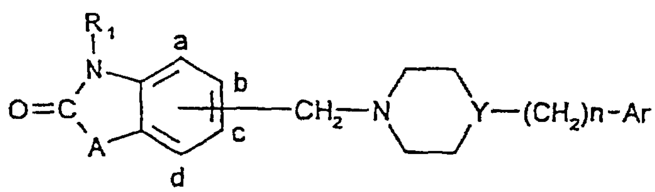

(I)

in der:

$R_1$ ein Wasserstoffatom oder eine Niedrigalkylgruppe darstellt oder
$R_1$ eine Gruppe

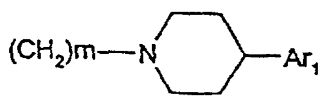

darstellt, in der m eine ganze Zahl zwischen 1 und 4 einschließlich und $Ar_1$

- entweder eine Gruppe $CO-Ar_2$, worin $Ar_2$ einen Phenylrest darstellt, der nicht substituiert oder durch eine oder mehrere Gruppen ausgewählt aus Halogen, Hydroxy, Niedrigalkyl, Trifluormethyl oder Niedrigalkoxy substituiert ist,
- oder eine Gruppe $=C-(Ar_2)_2$, worin $Ar_2$ die oben angegebenen Bedeutungen besitzt, bedeuten,

n 0 oder 1 darstellt,
A ein Sauerstoff- oder Schwefelatom darstellt,
Y eine Gruppe CH oder ein Stickstoffatom bedeutet und
Ar eine Phenyl- oder Naphthylgruppe, die gegebenenfalls durch eine, zwei oder drei Gruppen ausgewählt aus Halogen, Hydroxy, Niedrigalkoxy, Niedrigalkyl, Niedrigalkoxy-niedrigalkyl oder Trifluormethyl substituiert ist oder eine Aminosulfonylgruppe darstellt oder Ar eine Pyridyl-, Pyrimidinyl- oder 3-(Benzo[d]-1,2-thiazolyl)-gruppe, die auch als 3-Benzisothiazolylgruppe bezeichnet wird:

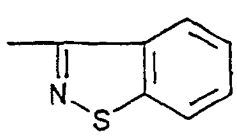

darstellt,

gegebenenfalls ihre Isomeren in reiner Form oder in Form einer Mischung sowie ihre Additionssalze mit einer pharmazeutisch annehmbaren Säure oder einer pharmazeutisch annehmbaren Base, wenn $R_1$ = H bedeutet, mit der Maßgabe, daß, wenn nichts anderes angegeben ist,

- die Begriffe "Niedrigalkyl" und "Niedrigalkoxy" geradkettigen oder verzweigten Gruppen, die 1 bis 6 Kohlenstoffatome enthalten, entsprechen.

**2.** Verbindungen der Formel (I) nach Anspruch 1, worin folgendes gemeinsam oder getrennt gilt:

. $R_1$ bedeutet ein Wasserstoffatom oder eine Methylgruppe oder eine Gruppe

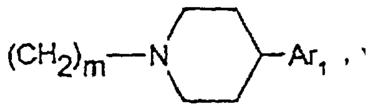

worin m den Wert 2 besitzt und $Ar_1$ eine Gruppe

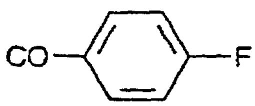

oder eine Gruppe

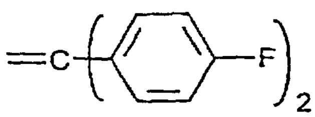

darstellt,

. A ein Schwefelatom darstellt,
. A ein Sauerstoffatom darstellt,
. die Seitenkette in der Position c gepfropft ist,
. n 0 bedeutet,
. Y ein Stickstoffatom bedeutet,
. Ar eine Phenylgruppe darstellt, die durch ein Fluoratom oder durch ein Chloratom oder eine Methoxygruppe substituiert ist, sowie deren Isomere in reiner Form oder in Form von Mischungen sowie ihre Additionssalze mit einer pharmazeutisch annehmbaren Säure oder einer pharmazeutisch annehmbaren Base, wenn $R_1$ = H ist.

**3.** Derivate der Formel (I) nach Anspruch 1, ausgewählt aus:

. 3-Methyl-6-{[4-(2-methoxyphenyl)-piperazin-1-yl]-methyl}-benzoxazolinon sowie dessen Additionssalze mit einer pharmazeutisch annehmbaren Säure,
. 3-Methyl-6-{[4-(2-methoxyphenyl)-piperazin-1-yl]-methyl}-benzothiazolinon sowie dessen Additionssalze mit einer pharmazeutisch annehmbaren Säure,
. 3-Methyl-6-{[4-(2-fluorphenyl)-piperazin-1-yl]-methyl}-benzothiazolinon sowie dessen Additionssalze mit einer pharmazeutisch annehmbaren Säure,
. 3-Methyl-6-{[4-(2-fluorphenyl)-piperazin-1-yl]-methyl}-benzoxazolinon sowie dessen Additionssalze mit einer pharmazeutisch annehmbaren Säure,
. 3-Methyl-6-{[4-(4-chlorphenyl)-piperazin-1-yl]-methyl}-benzoxazolinon sowie dessen Additionssalze mit einer pharmazeutisch annehmbaren Säure,
. 3-Methyl-6-{[4-(4-chlorphenyl)-piperazin-1-yl]-methyl}-benzothiazolinon sowie dessen Additionssalze mit einer pharmazeutisch annehmbaren Säure,

- 6-{[4-(2-Fluorphenyl)-piperazin-1-yl]-methyl}-benzoxazohnon sowie dessen Additionssalze mit einer pharmazeutisch annehmbaren Säure,
- 6-{[4-(2-Fluorphenyl)-piperazin-1-yl]-methyl}-benzothiazolinon sowie dessen Additionssalze mit einer pharmazeutisch annehmbaren Säure,
- 3-{2[4-(4-Fluorbenzoyl)-piperidyl]-ethyl}-6-{[4-(2 -fluorphenyl)-piperazin- 1-yl]-methyl}-benzoxazolinon sowie dessen Additionssalze mit einer pharmazeutisch annehmbaren Säure,
- 3-{2[4-(4',4"-Difluorbenzhydryliden)-piperidin-1-yl]-ethyl)-6-{[4-(2-fluorphenyl)-piperazin-1-yl]-methyl}-benzoxazolinon sowie dessen Additionssalze mit einer pharmazeutisch annehmbaren Säure,
- 3-{2[4-(4-Fluorbenzoyl)-piperidin-1-yl]-ethyl}-6-{[4-(2-fluorphenyl)-piperazin-1-yl]-methyl}-benzothiazolinon sowie dessen Additionssalze mit einer pharmazeutisch annehmbaren Säure,
- 3-Methyl-6-{[4-(benzo[d]1,2-thiazolyl)-piperazin-1-yl]-methyl}-benzoxazolinon sowie dessen Additionssalze mit einer pharmazeutisch annehmbaren Säure,
- 3-Methyl-6-{[4-(benzo[d]1,2-thiazolyl)-piperazin-1-yl)-methyl}-benzothiazolinon sowie dessen Additionssalze mit einer pharmazeutisch annehmbaren Säure,
- 3-Methyl-6-{[4-(4-aminosulfonylphenyl)-piperazin-1-yl]-methyl}-benzoxazolinon sowie dessen Additionssalze mit einer pharmazeutisch annehmbaren Säure,
- 3-Methyl-6-{[4-(4-aminosulfonylphenyl)-piperazin-1-yl]-methyl}-benzothiazolinon sowie dessen Additionssalze mit einer pharmazeutisch annehmbaren Säure,
- 3-Methyl-6-{[4-(3-methoxyphenyl) -piperazin- -yl]-methyl}-benzoxazolinon sowie dessen Additionssalze mit einer pharmazeutisch annehmbaren Säure,
- 3-Methyl-6-{[4-(4-methoxyphenyl)-piperazin-1-yl]-methyl}-benzothiazolinon sowie dessen Additionssalze mit einer pharmazeutisch annehmbaren Säure,
- 3-Methyl-6-{[4-(4-methoxyphenyl)-piperazin-1-yl]-methyl}-benzoxazolinon sowie dessen Additionssalze mit einer pharmazeutisch annehmbaren Säure.

**4.** Verbindung der Formel (I) nach Anspruch 1, nämlich 3-Methyl-6-{[4-(2-fluorphenyl)-piperazin-1-yl]-methyl}-benzothiazolinon sowie dessen Additionssalze mit einer pharmazeutisch annehmbaren Säure.

**5.** Verbindung der Formel (I) nach Anspruch 1, nämlich 3-Methyl-6-{[4-(4-chlorphenyl)-piperazin-1-yl]-methyl}-benzoxazolinon sowie dessen Additionssalze mit einer pharmazeutisch annehmbaren Säure.

**6.** Verfahren zur Herstellung der Verbindungen der Formel (I) nach Anspruch 1, dadurch gekennzeichnet, daß man

- einerseits, wenn in der herzustellenden Verbindung der Formel (I) die Gruppe $R_1$ eine Niedrigalkylgruppe darstellt, man eine Verbindung der Formel (II):

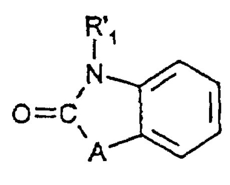

(II)

in der A die oben angegebenen Bedeutungen besitzt und $R'_1$ eine Niedrigalkylgruppe darstellt,
mit Hexamethylentetramin, vorzugsweise in saurem Medium, umsetzt zur Bildung eines Produkts der Formel (III):

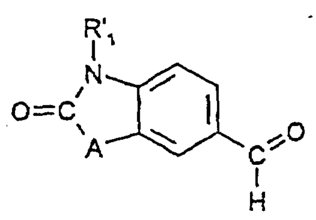

(III)

worin $R'_1$ und A die oben angegebenen Bedeutungen besitzen,

welches man mit einem Hydrierungsmittel behandelt zur Bildung eines Produkts der Formel (IV):

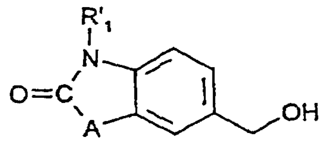

(IV)

worin $R'_1$ und A die oben angegebenen Bedeutungen besitzen,

. andererseits, wenn in der herzustellenden Verbindung der Formel (I) $R_1$ von einer Niedrigalkylgruppe verschieden ist, man ein Derivat der Formel (V):

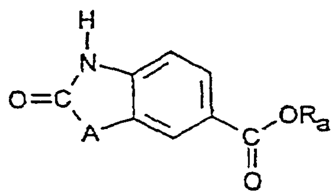

(V)

in der $R_a$ eine Niedrigalkylgruppe darstellt und A die oben angegebenen Bedeutungen besitzt, mit einem Hydriermittel behandelt zur Bildung eines Derivats der Formel (IVb):

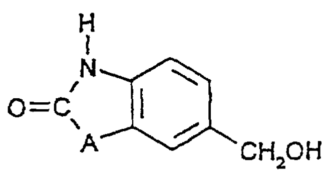

(IVb)

worin A die oben angegebenen Bedeutungen besitzt,

. man das Derivat der Formel (IV) oder (IVb) mit einem Halogenierungsmittel behandelt zur Bildung eines Produkts der Formel (VI):

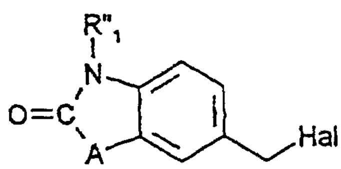

(VI)

worin $R''_1$ ein Wasserstoffatom oder eine Niedrigalkylgruppe darstellt, A die oben angegebenen Bedeutungen besitzt und Hal für ein Halogenatom steht, welches man mit einem Amin der Formel (VII) behandelt:

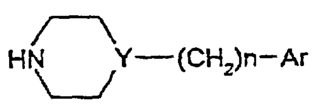

(VII)

worin Y, n und Ar die bezüglich der Formel (I) angegebenen Bedeutungen besitzen,
zur Bildung eines Produkts der Formel (I/a):

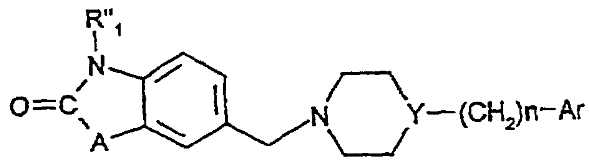

(I/a)

einem Sonderfall der Derivate der Formel (I), worin $R''_1$, A, Y, n und Ar die oben angegebenen Bedeutungen besitzen,
welches Derivat der Formel I/a, wenn in dem herzustellenden Derivat der Formel (I) $R_1$ weder ein Wasserstoffatom noch eine Niedrigalkylgruppe darstellt, mit einem Derivat der Formel (IX) behandelt wird:

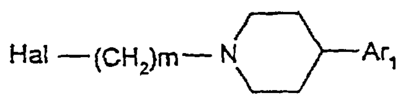

(IX)

worin Hai ein Halogenatom darstellt und m und $Ar_1$ die bezüglich der Formel (I) angegebenen Bedeutungen besitzen,
zur Bildung eines Derivats der Formel I/c:

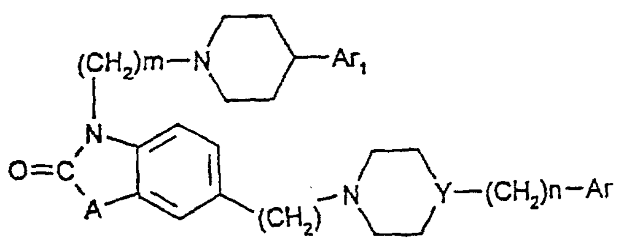

(I/c)

einem Sonderfall der Derivate der Formel (I), worin $R_1$ eine Gruppe

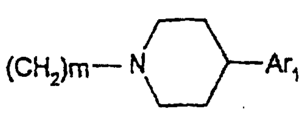

darstellt, welches in dieser Weise erhaltene Derivat der Formel I/a oder I/c man gegebenenfalls

- mit Hilfe einer oder mehrerer Methoden ausgewählt aus Kristallisation, Chromatographie, Extraktion oder Überführung über ein Harz oder Aktivkohle reinigt,
- man gegebenenfalls in seine optischen Isomeren in reiner Form oder in Form einer Mischung trennt,
- und gewünschtenfalls mit einer pharmazeutisch annehmbaren Säure oder Base in das Salz überführt,

wobei es sich versteht, daß der Begriff "Niedrigalkyl" für geradkettige oder verzweigte Gruppen, die 1 bis 6 Kohlenstoffatome enthalten, steht.

**7.** Verbindungen der Formeln III, IV, IVb und VI nach Anspruch 6 als Zwischenprodukte für die Synthese der Derivate der Formel (I) mit Ausnahme des Derivats der Formel III, worin A ein Sauerstoffatom und $R'_1$ eine Methygruppe bedeuten.

**8.** Verbindungen nach Anspruch 7, nämlich:

- 3-Methyl-6-formyl-benzothiazolinon,
- 3-Methyl-6-hydrorymethyl-benzoxazolinon,
- 3-Methyl-6-hydroxymethyl-benzothiazolinin,

- 6-Hydroxymethyl-benzoxazolinon,
- 6-Hydroxymethyl-benzothiazolinon,
- 3-Methyl-6-halogenomethyl-benzoxazolinone,
- 3-Methyl-6-halogenomethyl-benzothiazolinone,
- 6-Halogenomethyl-benzoxazolinone und
- 6-Halogenomethyl-benzothiazolinone.

**9.** Pharmazeutische Zubereitungen enthaltend als Wirkstoff mindestens eine Verbindung nach einem der Ansprüche 1 bis 5 in Kombination mit einem oder mehreren pharmazeutisch annehmbaren Trägermaterialien oder Bindemitteln.

**10.** Pharmazeutische Zubereitungen nach Anspruch 9 zur Verwendung bei der Behandlung von Störungen des psychischen Verhaltens und insbesondere von Psychosen, der Schizophrenie, Veränderungen der Stimmung und von Erkenntnisfunktionen, der Angst, von Depressionen, dem Drogenmißbrauch, von impulsiven Zuständen, Gedächtnis-Erkenntnis-Störungen und von Migränezuständen.